(19) **Europäisches Patentamt / European Patent Office / Office européen des brevets**

(11) **EP 2 521 547 B1**

(12) **EUROPEAN PATENT SPECIFICATION**

(45) Date of publication and mention of the grant of the patent:
**16.08.2017 Bulletin 2017/33**

(51) Int Cl.:
*A61K 9/06* (2006.01)          *A61K 31/437* (2006.01)
*A61K 47/10* (2017.01)        *A61K 47/36* (2006.01)
*A61K 47/38* (2006.01)        *A61K 47/30* (2006.01)
*A61P 27/16* (2006.01)

(21) Application number: **11732210.7**

(22) Date of filing: **07.01.2011**

(86) International application number:
**PCT/US2011/020531**

(87) International publication number:
**WO 2011/085209 (14.07.2011 Gazette 2011/28)**

(54) **METHODS AND COMPOSITIONS FOR APPLYING MOXIFLOXACIN TO THE EAR**

VERFAHREN UND ZUSAMMENSETZUNGEN ZUR EINFÜHRUNG VON MOXIFLOXACIN IN DAS OHR

PROCÉDÉS ET COMPOSITIONS POUR L'APPLICATION DE MOXIFLOXACINE DANS L'OREILLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.01.2010 US 293019 P**

(43) Date of publication of application:
**14.11.2012 Bulletin 2012/46**

(73) Proprietors:
• **Regents of the University of Minnesota**
  **St. Paul, Minnesota 55114-8658 (US)**
• **Novartis AG**
  **4056 Basel (CH)**

(72) Inventors:
• **SAWCHUK, Ronald J.**
  **Prior Lake**
  **Minnesota 55372 (US)**
• **CHEUNG, Belinda W.Y.**
  **New Brighton**
  **Minnesota 55112 (US)**
• **WALL, G. Michael**
  **Fort Worth**
  **Texas 76109 (US)**

(74) Representative: **Leonard, Thomas Charles**
  **Kilburn & Strode LLP**
  **20 Red Lion Street**
  **London WC1R 4PJ (GB)**

(56) References cited:
**EP-A1- 1 312 366          WO-A2-2009/142719**
**US-A1- 2002 022 629     US-A1- 2007 098 679**
**US-A1- 2007 212 343**

• **C. M. CÁRCELES ET AL: "Pharmacokinetics of Moxifloxacin in Rabbits After Intravenous, Subcutaneous and a Long-acting Poloxamer 407 Gel Formulation Administration", JOURNAL OF VETERINARY MEDICINE SERIES A, vol. 53, no. 6, 1 August 2006 (2006-08-01), pages 300-304, XP55102133, ISSN: 0931-184X, DOI: 10.1111/j.1439-0442.2006.00827.x**

## Description

### TECHNICAL FIELD

The invention relates to formulations for applying moxifloxacin to the ear. More particularly, the invention features methods and materials for applying moxifloxacin to the external, epidermal surface of a tympanic membrane for treating disorders of the middle ear.

### BACKGROUND

Otitis media (OM) is very common, especially in children. OM often begins with a viral infection of the upper respiratory tract that alters the micro-environment of the upper respiratory tract, Eustachian tube, and middle ear such that bacteria resident in the nasopharynx invade and populate the middle ear. This invasion can inflame and block the Eustachian tube, interfering with middle ear ventilation, pressure equilibration, and drainage. Fluids accumulate and pressure increases in the normally air-filled middle ear space, causing great pain. In severe cases of OM, sound perception structures can be damaged. Persistent or recurrent OM may be caused by bacteria that emerge from dormancy in the middle ear, having been shielded from antibiotics by a slimy biofilm.

OM currently is treated using antibiotics and/or by inserting a tympanostomy tube through a surgical incision in the tympanic membrane so as to drain and depressurize the middle ear space. The efficacy of antibiotic treatment is limited by the route of delivery. Antibiotics can be delivered systemically, but a high dose often is required to attain therapeutic levels (i.e., above minimum inhibitory concentration) in the middle ear, and such levels often are attained after a significant lag time. Antibiotics also can be delivered by lavage, or via drops into the ear canal. Such delivery routes can be difficult to control, and often are not effective to achieve prolonged therapeutic levels of antibiotic in the middle ear. Antibiotics also can be delivered by injection into the middle ear, or by inserting antibiotic-impregnated materials into the middle ear, but such methods involve piercing or cutting the tympanic membrane, which requires general anesthesia and can damage the tympanic membrane. Surgical insertion of tympanostomy tubes also carries risks, including tympanoclerosis (i.e., calcification of the tympanic membrane and middle ear tissues), hearing loss, persistent otorrhea (i.e., discharge of pus from the tube) and infection.

The National Institute on Deafness and Other Communication Disorders (NIDCD), a part of the National Institutes of Health, recently launched a $2,000,000 funding initiative to support the development of alternative strategies and new approaches for preventing and treating OM. In its request for applications (RFA-DC-02-002), NIDCD stated that: (1) OM causes significant childhood morbidity and is increasingly affecting general public health; (2) OM is the leading reason for Emergency Room visits; (3) OM is the second leading reason for doctors' office visits; (4) OM is the leading reason of childhood antibiotics prescriptions, accounting for more than 40% of all outpatient antibiotic prescriptions; (5) OM is the leading reason for childhood hearing loss; and (6) OM is the leading reason for general anesthesia in children. In addition, NIDCD blamed the use of broad-spectrum antibiotics to treat OM for the alarming emergence of multiple antibiotic resistant bacteria in three of the genera that can cause OM (*Streptococcus pneumoniae,* non-typeable *Haemophilus influenzae*, and *Moraxella catarrhalis*). As a consequence, many first and second line antibiotics are becoming less and less effective against OM and other diseases, including pneumonia and meningitis. NIDCD concluded that "the development of novel approaches for the study, treatment and prevention of OM is urgently needed to: 1) reduce OM morbidity and the associated costs; and 2) preserve the efficacy of antibiotics used for the treatment of OM and other common serious diseases." WO 2009/142719 A discloses *in situ* hydrogel formulations comprising ciprofloxacin for application to the tympanic membrane.

### SUMMARY

The subject matter for which protection is sought is as defined in the claims. The invention is based, in part, on the discovery that compositions containing moxifloxacin can be formulated such that that it can be delivered to the external, epidermal surface of the tympanic membrane in a liquid-like form, then, upon delivery, transform to a solid-like gel state such that the composition remains localized against the tympanic membrane. Delivery of such compositions to the tympanic membrane can provide more effective ways to treat middle and inner ear disorders (e.g., OM).

In one aspect, the invention provides a formulation for use by administration to the tympanic membrane, wherein said formulation is aqueous and comprises a viscogenic agent and moxifloxacin or a salt thereof; wherein said formulation is flowable and has a viscosity less than 100 Pa*s at 25°C; wherein said formulation, after application to said tympanic membrane, forms a gel that has a yield stress sufficient to maintain said formulation against said tympanic membrane; and wherein said moxifloxacin transfers across the tympanic membrane and into the middle ear space when the formulation is applied to the tympanic membrane.

The viscogenic agent can be gellan, N-isopropyl acrylamide with sodium acrylate and n-N-alkylacrylamide, polyacrylic acid with polyethylene glycol, polymethacrylic acid with polyethylene glycol, CARBOPOL® (polyacrylic acid) with hydroxypropylmethylcellulose, cellulose acetate hydrogen phthalate latex, sodium alginate, or a reverse thermosetting gel such as a poloxamer or a poloxamine. The moxifloxacin can transfer across the tympanic membrane into the middle ear space. The at least one pharmacologic agent can include an antibiotic and the formulation further can include an anti-inflammatory agent, an anesthetic, an adhesion facilitator, a permeability or penetration enhancer, a bioadhesive, a hygroscopic agent, an ear war softener, or a preservative.

In another aspect, the invention provides an aqueous formulation comprising a viscogenic agent, moxifloxacin or a salt thereof, and isopropyl myristate (IPM),

wherein said formulation is flowable and has a viscosity less than 100 Pa*s at 25°C;

wherein said formulation, after application to the tympanic membrane of the ear of a subject, forms a gel that has a yield stress sufficient to maintain said formulation against the tympanic membrane; and

wherein said moxifloxacin transfers across the tympanic membrane and into the middle ear space when the formulation is applied to the tympanic membrane.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. In case of conflict, the present specification, including definitions, will control. In addition, the materials, methods, and examples are illustrative only and not intended to be limiting.

Other features and advantages of the invention will be apparent from the following detailed description, and from the claims.

## DESCRIPTION OF DRAWINGS

FIG. 1 is a graph showing the cumulative percent of moxifloxacin content released *in vitro.*

FIG. 2 is a graph showing the response function for deconvolution from two intrabulla dose levels (N=9).

FIG. 3 is a graph showing the response function for deconvolution from high intrabulla dose level (N=3).

FIG. 4A are graphs showing the moxifloxacin concentrations in MEF following external ear dosing of 1% moxigel for cohort 1 and cohort 2.

FIG 4B are graphs showing the moxifloxacin concentrations in MEF following external ear dosing of 1% moxigel for cohort 3 and cohort 4.

FIG. 5 are graphs showing the moxifloxacin concentration (mean, SD) in MEF following external ear dosing of 1% moxigel (N=23).

FIG. 6 are graphs showing the moxifloxacin concentrations in MEF following external ear dosing of 3% moxigel for cohort 1 and cohort 2.

FIG. 7 are graphs showing the moxifloxacin concentrations (mean, SD) in MEF following external ear dosing of 3% moxigel (N=13).

FIG. 8 is a graph showing the moxifloxacin concentrations (mean, SD) in MEF following external ear dosing of 1% and 3% moxigel.

FIG. 9 are graphs showing a comparison of Cmax and Tmax for 1% and 3% moxigel.

FIG. 10 is a graph showing the cumulative amount delivered into MEF following external ear dosing of 1% moxigel (N=23).

FIG. 11A are graphs showing the input rate into MEF following external ear dosing of 1% moxigel. 1st cohort, N=6; 2nd cohort, N=6.

FIG. 11B are graphs showing the input rate into MEF following external ear dosing of 1% moxigel. 3rd cohort, N=6; 4th cohort, N=5.

FIG. 12 is a graph showing the cumulative amount delivered into MEF following external ear dosing of 3% moxigel (N=13).

FIG. 13 are graphs showing the input rate into MEF following external ear dosing of 3% moxigel. 1st cohort, N=6; 2nd cohort, N=7.

FIG. 14 is a graph showing the input rate into MEF following external ear dosing of 1% and 3% moxigel.

FIG. 15 are graphs showing time to reach and duration above target concentration in the middle ear fluid following external ear dosing with 1% moxigel.

FIG. 16 are graphs showing time to reach and duration above target concentration in the middle ear fluid following external ear dosing with 3% moxigel.

FIG. 17 is a graph showing the AUIC in MEF over 3 days following external ear dosing.

FIG. 18 is a graph showing the Cmax / MIC in MEF following external ear dosing.
FIG. 19A is a graph showing MEF moxifloxacin concentrations (Mean, SEM) following 50% IPM treatment (N = 9);
FIG. 19B shows the same data in log form.

## DETAILED DESCRIPTION

In general, the invention provides formulations for use by administration to the tympanic membrane, the formulations containing moxifloxacin and one or more viscogenic agents. The invention also provides a specific combination formulation additionally comprising isopropyl myristate (IPM) as a penetration enhancer. Compositions are specifically formulated such that they can be delivered to the external, epidermal surface of the tympanic membrane in a liquid-like state, i.e., a flowable form. After administration, however, the composition transforms into a solid-like state such that the composition remains in contact with the tympanic membrane. As a result, the composition remains localized against the tympanic membrane and the moxifloxacin can transfer across the tympanic membrane into, for example, the middle ear space, providing a more effective way to treat middle and inner ear disorders (e.g., OM). Suitable compositions also can contain other constituents, e.g., to facilitate the adhesion of the formulation to the tympanic membrane and/or to increase the permeability of the tympanic membrane to thereby increase the penetration of the moxifloxacin.

The common pathogens associated with otitis media include *Streptococcus pneumoniae*, *Haemophilus influenza* and *Moraxella catarrhalis.* These organisms are sensitive to moxifloxacin; having MIC90 values of 0.13, 0.06 and 0.06 $\mu$g/ml, respectively. As is understood by those skilled in the art, MIC90 refers to the Minimum Inhibitory Concentration required to inhibit the growth of 90% of the organism. The therapeutic goal using the transtympanic delivery of moxifloxacin described herein is to achieve middle ear fluid levels that are >10-fold higher than the MIC90 values (e.g., greater than about 0.6 $\mu$g/ml; between about 0.6 and about 1.3 $\mu$g/ml) and to sustain this level for >24 hours. These target concentrations have been achieved with the moxifloxacin gel formulations described herein using a single application.

Compositions of, and used in, the invention have a viscosity of less than 100,000 mPa*s at 25°C. Viscosity refers to the composition's resistance to flow. Compositions having a volume of 0.5 mL that can pass through a 19-gauge needle attached to a 1-mL tuberculin syringe in less than 1 minute at 25°C, by reasonable force and without aid of mechanical devices, typically have a viscosity of less than 100,000 mPa*s. Viscosity of a composition can be determined using a viscometer (e.g., from Brookfield) calibrated with commercially available viscosity standards.

Compositions of, and used in, the invention also have a minimum yield stress that is sufficient for maintaining the formulation against the tympanic membrane. Yield stress refers to the amount of force that, when applied to a solid material, causes the solid material to exhibit liquid-like behavior in that it continues to deform with no further increase in stress. Minimum yield stress of compositions of, or for use in, the invention is dependent on the thickness of the applied gel, but is independent of the geometry of the gel and the temperature of the environment. As used herein, minimum yield stress of the composition is in reference to an applied gel that has a thickness of 4 mm and a density of 1 g/L. Yield stress ($\sigma_0$) is represented as $\sigma_0 = \rho g h$, where $\rho$ is the density, $g$ is the acceleration due to gravity, and $h$ is the layer thickness. Typically, minimum yield stress is about 39 pascals (Pa). Methods described herein also can be used to estimate if a composition has sufficient yield stress to be maintained against the tympanic membrane. For example, a test composition can be administered to the ear of an animal such as a chinchilla and the ear of the animal can be monitored to determine if the composition transforms to a more solid-like state and is maintained against the tympanic membrane. See, for example, the Example section herein.

### Viscogenic Agents

As used herein, viscogenic agent refers to a polymer or other chemical moiety that increases the viscosity of a fluid. Suitable viscogenic agents, when included in a composition of, or for use in, the invention, allow the composition to transform from a liquid-like state (e.g., flowable) at 25°C to a solid-like state (e.g., a gel) after contact with the tympanic membrane, and can be non-biodegradable, i.e., not broken down by chemicals or enzymes naturally present in a mammal, or biodegradable. Compositions include an amount of viscogenic agent effective to yield a viscosity of the composition of less than 100,000 mPa*s at 25°C (e.g., less than 90,000, 60,000, 30,000, 20,000, or 10,000 Pa*s) and, generally, a minimum yield stress of 39 Pa after application to the tympanic membrane. Typically, a composition includes 0.05 to 50% of a viscogenic agent (e.g., 0.15 to 25, 5 to 45, 10 to 40, 12 to 37, 15 to 35, 17 to 33, or 20 to 30% of a viscogenic agent).

Exemplary viscogenic agents include gellan (GELRITE® or KELCOGEN®), CARBOPOL® 940 (polyacrylic acid) with hydroxypropylmethylcellulose (HPMC), N-isopropyl acrylamide (NiPAAm) with sodium acrylate and n-N-alkylacrylamide, polyacrylic acid with polyethylene glycol (PEG) or polymethacrylic acid with PEG, cellulose acetate hydrogen phthalate latex (CAP), sodium alginate, and nonionic surfactants such as poloxamers (PLURONIC®) and polyoxamine (TETRONIC®) reversible temperature-dependent gelling systems. Gellan is a natural polymer, anionic deacetylated exocellular polysaccharide, secreted by *Pseudomonas elodea.* The tetrasaccharide repeating unit consists of one alpha-L-rhamnose, one beta-D-glucuronic acid, and two beta-D-glucose moieties. The *in situ* gelling mechanism of gellan is cation-induced (e.g., presence of calcium ions) and temperature-dependent (e.g., physiologic temperature). Gelation is thermally reversible. CARBOPOL® 940 with HPMC gels *in situ* in a pH-dependent manner. CARBOPOL® is the gelling agent and the HPMC is used to enhance the viscosity of the gel. NiPAAm with sodium acrylate and n-N-alkylacrylamide is a terpolymer hydrogel that can undergo a temperature based reversible sol-gel transformation. Sodium acrylate and n-N-alkylacrylamide are used to modify the properties of the hydrogel, and in particular, the transition temperature. Polyacrylic acid with PEG or polymethacrylic acid with PEG is thought to gel based on hydrogen bonding. Polyacrylic acid can be dissolved in hydroalcoholic solution and after being injected, the alcohol diffuses out causing the polymers to precipitate and gelling of the solution. CAP is a nanoparticulate system that gels in a pH-dependent manner. The active compound (moxifloxacin) may be adsorbed partially onto the surface of the polymer particles. Sodium alginate gels in the presence of calcium or other polyvalent ions.

Nonionic surfactants such as poloxamers and poloxamines are particularly useful. Poloxamers are well known in the pharmaceutical arts and are described, for example, by Irving R. Schmolka, Poloxamers in the Pharmaceutical Industry, in Polymers for Controlled Drug Delivery, Chapter 10 (Peter J. Tarcha ed., 1990). Poloxamers are triblock copolymers because they are composed of two different polymer blocks (i.e., hydrophilic poly(oxyethylene) blocks and hydrophobic poly(oxypropylene) blocks) configured as a triblock of poly(oxyethylene)-poly(oxypropylene)-poly(oxyethylene). Poloxamers are one class of block copolymer surfactants having a propylene oxide block hydrophobe and an ethylene oxide hydrophile. Poloxamers are commercially available (e.g., PLURONIC® polyols are available from BASF Corporation). Alternatively, polaxamers can be synthesized by known techniques.

Poloxamers previously have been thought to lack utility for administering pharmacologic agents, given their non-biodegradability, their water solubility and their relatively rapid drug release kinetics (see e.g., U.S. Patent No. 6,201,072). Nonetheless, as described herein, poloxamers share a property that is advantageous for applying formulations to the tympanic membrane: aqueous formulations of poloxamers exhibit reverse thermal gelation, or reverse thermosetting. When an aqueous poloxamer formulation is heated over its gelation temperature, its viscosity increases and it transforms into a gel. When an aqueous poloxamer formulation is cooled below its gelation temperature, its viscosity decreases and it transforms into a liquid. The transition between gel and liquid does not involve a change in the chemical composition of the formulation, and is reversible and repeatable. The gel-liquid transition temperature of an aqueous poloxamer formulation can be adjusted by one of ordinary skill in the art using routine experimentation (e.g., by manipulating poloxamer concentration, pH and presence of other ingredients in the formulation). In some embodiments, compositions have a gelation temperature that is greater than the ambient temperature and less than or equal to the temperature of the tympanic membrane. Such compositions can be conveniently applied via an individual's ear canal as a liquid and then can transform into a gel against the tympanic membrane, thereby maintaining the moxifloxacin in the formulation in close proximity to the tympanic membrane.

*Moxifloxacin*

A composition as described herein also contains moxifloxacin or a salt thereof. Moxifloxacin is a third generation synthetic fluoroquinolone having the chemical formula $C_{21}H_{24}FN_3O_4$. Moxifloxacin binds to and inhibits the bacterial enzymes DNA gyrase (topoisomerase II) and topoisomerase IV, resulting in inhibition of DNA replication and repair and ultimately cell death in sensitive bacterial species. The amount of moxifloxacin or salt thereof in a composition as described herein can range from about 0.1% to about 50% (e.g., about 0.25% to about 45%; about 0.5% to about 25%; about 0.75% to about 10%; about 1% to about 5%; or about 1% to about 3%). Salts of moxifloxacin include, for example and without limitation, hydrochloric acid, sulfuric acid, acetic acid, lactic acid, sodium hydroxide, and potassium hydroxide.

*Other Constituents of Moxigel Compositions*

In some embodiments, compositions as described herein include one or more compounds in addition to the viscogenic and moxifloxacin. For example, a composition can include one or more pharmacological agents, including, e.g., adrenocorticoid (e.g., corticosteroid, steroid), analgesic, analgesic adjunct, analgesic-anesthetic, anesthetic, antibiotics other than moxifloxacin, antibacterial, anti-infective, antibiotic therapy adjunct, antidote, anti-emetic, anti-fungal, anti-inflammatory, anti-vertigo, anti-viral, biological response modifier, cytotoxic, diagnostic aid, immunizing agent, immunomodulator, proteins, peptides, and other agents that may be useful in treating ear disorders. In addition to moxifloxacin, a composition as described herein can include one or a plurality of pharmacological agents. For example, to fight a bacterial infection, to reduce tissue inflammation, and to alleviate irritation, a composition can contain moxifloxacin, an anti-inflammatory, and an anesthetic or analgesic. Those skilled in the art can identify pharmacological agents and combine them as needed to achieve a desired effect. The following simply provides a representative list of possible pharmacological agents.

Exemplary adrenocorticoids include betamethasone, cortisone, dexamethasone, hydrocortisone, methylprednisolone, paramethasone, prednisolone, prednisone, and triamcinolone. Exemplary analgesics include acetaminophen, aspirin, buprenorphine, butalbital, butorphanol, codeine, dezocine, diflunisal, dihydrocodeine, etodolac, fenoprefen, fentanyl, floctafenine, hydrocodone, hydromorphone, ibuprofen, ketoprofen, ketorolac, levorphanol, magnesium salicylate, meclofenamate, mefenamic acid, meperidine, meprobamate, methadone, methotrimeprazine, morphine, nalbuphine, naproxen, opium, oxycodone, oxymorphone, pentazocine, phenobarbital, propoxyphene, salsalate, and sodium salicylate. One exemplary analgesic adjunct is caffeine. Exemplary anesthetics include articaine-epinephrine, bupivacaine, chloroprocaine, etidocaine, ketamine, lidocaine, mepivacaine, methohexital, prilocaine, propofol, propoxycaine, tetracaine, and thiopental. One exemplary analgesic-anesthetic is antipyrine-benzocaine.

Exemplary antibiotics (other than moxifloxacin), anti-bacterials, and anti-infectives include sulfonamides (e.g., sulfanilamide, sulfadiazine, sulfamethoxazole, sulfisoxazole, para-aminobenzoic acid, or sulfacetamide), trimethoprim-sulfamethoxazole, quinolones (e.g., ciprofloxacin, ofloxacin, or nalidixic acid), beta-lactam antibiotics such as penicillins or cephalosporins, aminoglycosides (e.g., kanamycin, tobromycin, gentamycin C, amikacin, neomycin, netilmicin, streptomycin, or vancomycin), tetracyclines, chloramphenicol, and macrolides (e.g., erythromycin, clarithromycin, or azithromycin). Non-limiting examples of suitable penicillins include penicillin G, penicillin V, methicillin, oxacillin, nafcillin, ampicillin, and amoxicillin. Non-limiting examples of suitable cephalosporins include cephalothin, cefdinir, cefazolin, cephalexin, cefadroxal, cefamandole, cefoxitin, cefaclor, cefonicid, cefoletan, cefotaxime, ceftizoxime, ceftriaxone, cefditoren, and cefepime. Exemplary antibiotics useful for treating OM include penicillins such as amoxicillin and amoxicillin-clavulanate (AUGMENTIN®); sulfa-based combinations such as erythromycin-sulfisoxazole (Pediazole), trimethoprim-sulfamethoxazole (BACTRIM®, SEPTRA®); macrolides / azalides such as azithromycin (ZITHROMAX®) or clarithromycin (BIAXIN®); second-generation cephalosporins such as cefaclor (CECLOR®), cefprozil (CEFZIL®), cefuroxime axetil (CEFTIN®), or loracarbef (LORABID®); and third generation cephalosporins such as cefdinir (OMNICEF®), cefixime (SUPRAX®), cefpodoxime proxetil (VANTIN®), ceftibuten (CEDAX®), cefditoren (SPECTRACEF™), and ceftriaxone (ROCEPHIN®).

Suitable anti-emetics include buclizine, chlorpromazine, cyclizine, dimenhydrinate, diphenhydramine, diphenidol, domperidone, dronabinol, haloperidol, hydroxyzine, meclizine, metoclopramine, nabilone, ondansetron, perphenazine, prochlorperazine, promethazine, scopolamine, thiethylperazine, triflupromazine, and trimethobenzamine. Exemplary antifungals include amphotericin B, clioquinol, clotrimazole, fluconazole, flucytosine, griseofulvin, ketoconazole, miconazole, and potassium iodide. Exemplary anti-inflammatory agents include aluminum acetate, aspirin, betamethasone, bufexamac, celecoxib, dexamethasone, diclofenac, etodolac, flurbiprofen, hydrocortisone, indomethacin, magnesium salicylate, naproxen, prednisolone, rofecoxib, salsalate, sulindac, and triamcinolone. Exemplary suitable anti-vertigo agents include belladonna, dimenhydrinate, diphenhydramine, diphenidol, meclizine, promethazine, and scopolamine. Exemplary suitable anti-viral agents include acyclovir, amantadine, delavirdine, didanosine, efavirenz, foscarnet, ganciclovir, indinavir, nelfinavir, ribavirin, ritonavir, zalcitabine, and zidovudine. Exemplary biological response modifiers include aldesleukin, interferon α-2a, interferon α-2b, interferon α-n1, interferon α-n3, interferon γ, and levamisole. Exemplary cytotoxic agents include podofilox and podophyllum. Exemplary immunizing agents include influenza virus vaccine, pneumococcal vaccine polyvalent, and immune globulin. An exemplary immunomodulator is interferon γ. Other pharmacological agents suitable for, or for use in, the invention include betahistine (e.g., for treating the nausea, dizziness, and ringing in the ears that occur in Ménière's disease), prochlorperazine, and hyoscine.

Alternatively or additionally, a composition can include one or more of the following compounds: a solvent or diluent such as saline, a bioadhesive, a permeability or penetration enhancer, a hygroscopic agent, an earwax softener, preservative (e.g., an antioxidant), or other additives. Such compounds can be present in the composition in amounts ranging from 0.01% to 99% (e.g., 0.01 to 1, 0.01 to 10, 0.01 to 40, 0.01 to 60, 0.01 to 80, 0.5 to 10, 0.5 to 40, 0.5 to 60, 0.5 to 80, 1 to 10, 1 to 40, 1 to 60, 1 to 80, 5 to 10, 5 to 40, 5 to 60, 5 to 80, 10 to 20, 10 to 40, 10 to 60, 10 to 80, 20 to 30, 30 to 40, 40 to 50, 50 to 60, 60 to 70, or 70 to 80%). For example, a composition can include one or more viscogenic agents (e.g., PLURONIC® F-127 and CARBOPOL®), moxifloxacin, and one or more permeability or penetration enhancers (e.g., vitamin E). In other embodiments, a composition can include one or more viscogenic agents, moxifloxacin, and one or more earwax softeners. Compositions also can include one or more viscogenic agents, moxifloxacin, one or more hygroscopic agents, and one or more preservatives. It is noted that certain agents can fulfill different roles within the formulation. For example, CARBOPOL® can function as a viscogenic agent or as a bioadhesive, depending on its concentration. Vitamin E can function as a permeability or penetration enhancer, a preservative, and an antioxidant.

A bioadhesive facilitates the adhesion of the composition to the tympanic membrane. Suitable bioadhesives include hydrocolloids such as: acacia; agar agar; alginates (e.g., alginic acid and sodium alginate); CABOPOL®; carboxymethylcellulose sodium; carboxymethylcellulose calcium; dextran; gelatin; guar gum; heparin; hyaluronic acid; hydroxyethylcellulose; karaya gum; methylcellulose; pectin; polyacrylic acid; polyethylene glycol; poly-N-vinyl-2-pyrrolidone; and tragacanth.

Permeability or penetration enhancers increase the permeability of the tympanic membrane to make it more permeable to the moxifloxacin. Exemplary permeability or penetration enhancers include: alcohols (e.g., ethanol and isopropanol); polyols (e.g., n-alkanols, limonene, terpenes, dioxolane, propylene glycol, ethylene glycol, and glycerol); sulfoxides (e.g., dimethylsulfoxide, dimethylformamide, methyl dodecyl sulfoxide, and dimethylacetamide); esters (e.g.,

isopropyl myristate/palmitate, ethyl acetate, butyl acetate, methyl proprionate, and capric/caprylic triglycerides); ketones; amides (e.g., acetamides); oleates (e.g., triolein); surfactants (e.g., sodium lauryl sulfate); alkanoic acids (e.g., caprylic acid); lactams (e.g., azone); alkanols (e.g., oleyl alcohol); dialkylamino acetates; polyunsaturated fatty acids (e.g., linoleic, alpha-linolenic, and arachidonic); oleic acid; cod-liver-oil; menthol derivatives (e.g., 1-menthol); Squalene; glycerol monoethers derived from linear saturated fatty alcohols; flavones (e.g., chamomile apigenin, luteolin, and apigenin 7-O-beta-glucoside); vitamin E ($\alpha$-tocopherol) and esters and analogs thereof; and Senkyu (Ligustici Chuanxiong Rhizome) ether extract.

Hygroscopic agents such as fructose, phthalic acid, and sorbitol, facilitate the transfer of fluid from the middle ear across the tympanic membrane into the gel matrix. Hygroscopic agents can help alleviate pain associated with fluid accumulation and pressurization of the middle ear, and can concentrate the moxifloxacin in a smaller fluid volume in the middle ear.

Earwax softeners (e.g., docusate, olive oil, sodium bicarbonate, urea, or hydrogen peroxide) facilitate contact between the tympanic membrane and the composition. An antioxidant such as ascorbic acid and benzoic acid or other preservatives can be used to extend the shelf life of the formulation during storage.

*Methods of Applying a Composition to the Tympanic Membrane*

A composition of, or used in, the invention can be applied to the epidermal surface of a tympanic membrane via the external auditory canal to, for example, treat a middle or inner ear disorder (e.g., OM). Compositions of, or used in, the invention also can be applied prophylactically (e.g., to prevent the development of a middle or inner ear disorder). A composition can be targeted to any part of the tympanic membrane, including the *pars tensa*, the lower part of the tympanic membrane, or *pars flaccida,* the upper part of the tympanic membrane. In adult humans, the tympanic membrane is about nine to ten mm in diameter and has a thickness ranging from 30 to 230 $\mu$m (about 100 $\mu$m on average). The *pars flaccida* makes up less than 3% of the tympanic membrane area in humans and animals such as cats, guinea pigs, and chinchillas. In other mammals (e.g., gerbils, rabbits, rats, and mice), the *pars flaccida* makes up 10% to 25% of the tympanic membrane area. A thin epidermal layer (approximately 15 to 30 $\mu$m thick) covers the human tympanic membrane, while a thick epidermal layer (approximately 75 to 150 $\mu$m thick) covers other areas of the human body. Five to ten layers of cells cover the *pars flaccida,* while three to five layers of cells cover the *pars tensa.* Thus, the *pars tensa* often is thinner than other parts of the tympanic membrane and may be more permeable to the moxifloxacin or another pharmacological agent. It would be understood by those in the art that the central portion of the *pars tensa* provides the active vibrating area in response to sound.

Any method known in the art can be used to apply a composition of, or used in, the invention to the tympanic membrane. For example, a composition can be applied to the tympanic membrane using a fluid dispensing device. A dispensing device typically has a reservoir coupled to a conducting tube that directly or indirectly receives a flowable composition from the reservoir and conducts the composition to a dispensation outlet. One of ordinary skill can make a simple dispensing device as a matter of routine from a syringe connected to flexible tubing. A dispensing device also can be made by replacing the needle of a tympanocentesis device such as the CDT® Speculum (Walls Precision Instruments LLC, Casper, WY, USA) with a fluid conducting tube. A dispensing device can be attached to a pneumatic or diagnostic otoscope head (e.g., from Welch Allyn®, Skaneateles Falls, NY, USA) to create a precise platform for applying a composition to the tympanic membrane.

Depending on the composition and the middle or inner ear disorder, it may be desirable to remove the composition from the ear after the moxifloxacin has been transferred across the tympanic membrane. This can be accomplished manually using a cotton swab or forceps. A syringe or bulb also can be used to inject water, saline or other biocompatible aqueous solutions to soften, dissolve and / or flush out the formulation. In other embodiments, compositions simply may slough off the tympanic membrane after a period of time and fall out of the ear (e.g., during exercise or bathing). Alternatively, biodegradable formulations may not need to be removed from the ear.

*Articles of manufacture*

Compositions described herein can be combined with packaging material and sold as articles of manufacture or kits. Components and methods for producing articles of manufactures are well known. The articles of manufacture may combine one or more compositions described herein. In addition, the articles of manufacture may further include one or more pharmacological agents, sterile water or saline, pharmaceutical carriers, buffers, or fluid-dispensing devices. A label or instructions describing how the composition can be delivered to the ear for treatment of inner or middle ear disorders may be included in such kits. The compositions may be provided in a pre-packaged form in quantities sufficient for single or multiple administrations.

The invention will be further described in the following examples, which do not limit the scope of the invention described in the claims.

## EXAMPLES

Example 1-Chemicals and Reagents

Moxifloxacin hydrochloride powder was provided by Alcon Labs, Inc. (Fort Worth, TX). Pluronic F-127, ciprofloxacin hydrochloride, bovine albumin, formic acid, isopropyl myristate, and ammonium phosphate monobasic were purchased from Sigma-Aldrich (St. Louis, MO). The following chemicals were purchased and used as received: acetonitrile and methanol from Burdick and Jackson Laboratories (Muskegon, MI) or Fisher Scientific (Fair Lawn, NJ); sodium chloride from Mallinckrodt, Inc. (Paris, Kentucky); sodium phosphate dibasic, heptahydrate from United States Biochemical (Cleveland, OH); sodium phosphate monobasic from Fisher Scientific (Fair Lawn, NJ); propylene glycol from the University of Minnesota (Minneapolis, MN); PEG 4000 and PEG 6000 from Ruger Chemical Co, Inc. (Linden, NJ). Solvents were HPLC grade, and all other chemicals were analytical grade.

Example 2-Microdialysis Probes

CMA/20 microdialysis probes (CMA/Microdialysis, North Chelmsford, MA) were used to obtain the middle ear fluid dialysate samples. The polycarbonate membrane of the probe has a molecular weight cutoff of 20,000 Dalton. The length of the dialysate membrane of the probe was 10 mm. The outer diameter of the probe membrane was 0.5 mm.

Example 3-Artificial Middle Ear Fluid (AMEF)

The volume of serous middle ear fluid (MEF) that accumulates in non-infected chinchillas with Eustachian tube obstruction varies greatly. The success rate of accumulating enough MEF for microdialysis is significantly less than 50%. It became necessary to formulate an artificial middle ear fluid (AMEF) that mimics the MEF. For gel formulation dosing to the external ear, a phosphate buffered saline solution (PBS, 0.015 M phosphate, pH = 7.4) was instilled with 3% bovine albumin into the chinchilla middle ear bulla immediately prior to probe implantation.

Example 4-Validation of Ciprofloxacin as a Retrodialysis Calibrator of Moxifloxacin

Ciprofloxacin is a chemical analog of moxifloxacin and both are quinolone antibiotics. Ciproflaxin has physical and chemical properties somewhat similar to those of moxifloxacin. Therefore, it was selected as a potential retrodialysis calibrator. An *in vitro* simultaneous loss into AMEF study was conducted at perfusion flow rates of 0.4, 0.5, 0.6 and 0.7 μl/min with dialysate collection interval of 10 minutes. The perfusate contained 1 μg/ml of moxifloxacin and ciprofloxacin. The loss of each compound was determined by subtracting the ratio of the concentration in the dialysate to that in the perfusate from unity. It was found that the *in vitro* loss of moxifloxacin was not significantly different from that of ciprofloxacin across all 4 flow rates, validating the utility of ciprofloxacin as a microdialysis calibrator.

Example 5-Determination of the Free (Unbound) Fraction of Moxifloxacin in Artificial and Incubated Chinchilla Middle Ear Fluid

Previous in-house studies have shown that the artificial middle ear fluid (AMEF) began to resemble natural MEF and supported bacterial growth 18 to 24 hours after instillation into the chinchilla middle ear. For the purpose of determining the free fraction, the fluid was harvested from the middle ear following instillation of AMEF on the previous day and this fluid was referred to as incubated middle ear fluid (IMEF).

The protein binding of moxifloxacin in AMEF and IMEF was determined by ultrafiltration. The study was conducted at physiologic temperature (37°C) with nominal concentrations of 220 and 492 μg/ml. An aliquot of 120 μl of the spiked AMEF or IMEF solution was placed in the top portion of the Microcon® Centrifugal Device (Millipore Corporation, Bedford, MA) and centrifuged using a Clay Adams Triac 0200 swinging-bucket rotor centrifuge (Becton, Dickinson and Company, Parsippany, NJ) at 2000 xg for 15 to 20 minutes. To examine the extent, if any, of non-specific binding of moxifloxacin to the Microcon® device, the same drug concentrations were spiked in phosphate buffered saline and centrifuged through the device similar to the MEF aliquots. The ratio of the concentration in the ultrafiltrate to the total concentration in the MEF was calculated as the free fraction for moxifloxacin.

Example 6-Formulation Development

*Base Formulation*

Pluronic® F-127 (PF-127) was chosen as the polymer to form the thermosetting gel base. PF-127 dissolves in water to form solutions at lower temperatures and gels when the temperature increases above the sol-gel transition temperature. PF-127 solutions flow very well at room temperature and follow the contours of the surface onto which it is applied. The initial formulations studied are comprised of 20% (w/v) PF-127 in water.

PF-127 solution was prepared by the cold method. A weighed quantity of PF-127 was added slowly to the required amount of cold water (by weight), stirred gently and stored overnight at 4°C to allow the polymer to fully hydrate and dissolve.

*Transition Temperature Determination*

A small aliquot (100 to 200 μl) of the final formulation was transferred to a microcentrifuge tube. The tube was then incubated in a water bath at room temperature. The temperature of the water bath was increased gradually. Gelation was said to have occurred when the meniscus of the gel solution in the microcentrifuge tube was not distorted when tilted at a 90° or more angle. The transition temperature was taken as the two temperature values between which gelation occurred. The transition temperature of the base formulation (20% PF-127) was 22 to 23°C. The target transition temperature for the final formulation was between 28°C and 32°C.

*Additives*

Several additives were tested to assess their effects on the transition temperature and other properties of the gel - these include ethanol, propylene glycol, polyethylene glycol 4000 (PEG 4000), PEG 6000, and isopropyl myristate (IPM). Only PEG 6000 and ethanol were found to increase the transition temperature of the gel base significantly. Tables 1 and 2 show the effect of PEG 6000 and ethanol on the transition temperature of 20% PF-127 solution.

Table 1. Transition Temperatures of 20% PF-127 Solutions Containing Various Concentrations of PEG 6000

| PEG 6000 concentration | Transition temperature (°C) |
| --- | --- |
| 0.10% | 22 - 23 |
| 0.30% | 22 - 23 |
| 0.50% | 22 - 23 |
| 1% | 22 - 23 |
| 2% | 23 - 24 |
| 3% | 26 - 27 |
| 5% | >37 (failed to gel) |

Table 2. Transition Temperatures of 20% PF-127 Solutions Containing Various Concentrations of Alcohol and 2% PEG 4000

| Ethanol concentration | Transition temperature (°C) |
| --- | --- |
| 2% | 30 - 31 |
| 4% | 31 - 32 |
| 6% | 35 - 36 |
| 8% | >37 |

Higher concentrations of PF-127 (21 and 22%) were also tested but these formulations took an extended period of time to fully hydrate (up to 3 days) and were difficult to handle due to high viscosities. They also exhibited transition temperatures far below room temperature, ranging from subzero temperature for the base gel to less than 10°C following the addition of PEG 6000 for the 22% gel, and 23 to 24°C for the 21% gel.

The most promising base gel formulation developed has a transition temperature of 23 to 24°C, good flowability at room temperature, and has the following composition:

| | |
| --- | --- |
| PEG 6000 | 2% (v/w) |
| Propylene glycol | 20% (v/w) |
| PF-127 | 20% (w/w) |

Two levels of moxifloxacin hydrochloride were incorporated into the base gel: 1 and 3% (w/w). The 1% moxigel is a clear solution with a bright yellow color, while the 3% moxigel is a fine suspension with a white powder dispersed in the yellow liquid. Both moxigels exhibit a final transition temperature range of 29 to 31 °C.

To prepare a 10-ml batch of 1% moxigel, the following procedures were established:

1. To a scintillation vial, add 5.0 ml of PEG 6000 in water solution (40 mg/ml);
2. Add 2.0 ml of propylene glycol;
3. Slowly add 100 mg of moxifloxacin hydrochloride;
4. Sonicate the mixture for 3 to 5 minutes;
5. Add 1.3 ml of water;
6. Add 2 grams of PF-127;
7. Gently swirl the scintillation vial to wet the PF-127 powder;
8. Store the mixture in the refrigerator overnight for complete hydration; and
9. Vortex the solution thoroughly before use.

The procedures for the preparation of 3% moxigel are almost identical to those for 1% moxigel except that 300 mg of moxifloxacin hydrochloride is added and only 1.1 ml of water is required.

*In Vitro Release Studies*

The purpose of studying *in vitro* release of moxifloxacin was to determine the rate and extent of release from the gel formulations. For each release study, Franz diffusion cells were set up in a Hanson Microette apparatus (Hanson Research, Chatsworth, CA). The receiver compartments were filled with 8.0 ml of the release medium, 15% PF-127 in water that was magnetically stirred at 250 rpm. The temperature was maintained at 37°C with a water circulation jacket surrounding the lower portion of each Franz cell. Cellulose dialysis membrane sheets (Scienceware®, MW cutoff = 6 kD, Bel-Art Products, Pequannock, NJ, USA) were cut into small circles (3 cm in diameter) and mounted on top of each receiver compartment. A doughnut-shaped Teflon disk was placed on top of the membrane to form a donor compartment. An aliquot of 350 $\mu$l of each moxifloxacin gel formulation was then loaded into the dosing compartment. A waiting time of 5 min was allowed before sealing the Franz cells to ensure complete gel formation. The sample arm was covered with film to prevent evaporation. Each sample of 40 $\mu$l was withdrawn from the receiver compartment at 0.5, 1, 2, 3, 4, 6, 12, 24, 36, 48, 60, 72, 84 and 96 hours after gel administration. The samples were stored at -20°C until analysis.

Moxifloxacin concentrations in *in vitro* samples were determined by an offline HPLC-fluorescence method as outlined herein. To a 10 $\mu$l aliquot of each sample, 125 $\mu$l of phosphate buffered saline (PBS) was added to achieve equal volumes with the standard curve samples. Internal standard was then added (65 $\mu$l of 100 $\mu$g/ml ciprofloxacin) before being diluted to a final volume of 5.0 ml using mobile phase. Moxifloxacin standard samples of 0.05 to 20.0 $\mu$g/ml were prepared from the 1000, 100, 10, and 1 $\mu$g/ml standard stock solutions in PBS. To each standard curve sample, 65 $\mu$l of internal standard, 10 $\mu$l of 15% PF-127, and varying volume of PBS were added to achieve equal volumes with the *in vitro* samples before being diluted to a final volume of 5.0 ml using mobile phase. The mixture was vortexed at high speed for 30 sec. The injection volume was 50 $\mu$l.

Example 7-Surgery

*Animals*

Male *Chinchilla laniger* (Ryerson, Plymouth, OH or Dan Moulton, Rochester, MN), 390 - 670 g, were used in this study. The protocol was approved by the Institutional Animal Care and Use Committee (IACUC) at the University of Minnesota.

*Eustachian Tube Obstruction (ETO)*

The purpose of the ETO procedure is to prevent artificial middle ear fluid from draining into the nasopharynx to ensure that adequate fluid remained in the bulla during microdialysis. The middle portion of a gutta percha point (size 15, DiaDent®, DiaDent Group International Inc., Korea) was cut to a length of 4 mm and used to obstruct the Eustachian tube.

The ETO surgery was performed according to Jossart et al. (Jossart et al., 1990, Pharm. Res., 7:1242-7) with modification. Briefly, the animals were anesthetized with ketamine (40 to 50 mg/kg, IM) and pentobarbital (5 to 10 mg/kg, IP). A small incision was then made in the soft palate to expose the Eustachian tubes. The opening of each of the Eustachian tubes was obstructed with a 4 mm segment of the point. At the end of the surgery, the incision was closed using tissue adhesive (Vetbond®, 3M, St. Paul, MN).

*Artificial Middle Ear Fluid Instillation*

Instillation of artificial middle ear fluid (AMEF) into each bulla was performed the day of dosing prior to microdialysis probe implantation. Access to the chinchilla middle ear cavity was through the cephalad bulla on the dorsal side of the skull. A small hole was drilled manually with a 15 GA 1½ B hypodermic needle (Sherwood Medical Company, St. Louis, MO) at the apex of the right and left bulla where the bone is thin. Adequate AMEF was instilled into each bulla via a length of PE-50 tubing until it was completely filled to the top. The integrity of the tympanic membrane was examined using an otoscope. Ears with evidence of AMEF leakage into the external ear canal were not dosed.

*Implantation of Microdialysis Probes*

Implantation of microdialysis probe immediately followed AMEF instillation. Access to the chinchilla middle ear cavity was through the same hole on the cephalad bulla. A CMA/20 microdialysis probe was then carefully inserted into each middle ear cavity through the hole. The probes were secured onto the chinchilla skull according to the method developed by Huang et al. (Huang et al., 2001, J. Pharm. Sci., 90:2088-98) using a plastic crown secured by dental cement and anchor needles.

Example 8-Analytical Methods

*On-Line HPLC-MS-MS Analysis of Moxifloxacin and Ciprofloxacin in Microdialysates*

An on-line microdialysis HPLC-MS-MS system was established. Microdialysis perfusion flow rates were controlled with a Harvard microinjection pump (Model 22; Harvard Apparatus Inc.; South Natick, MA) fitted with 1-ml, 2.5-ml (CMA/Microdialysis, North Chelmsford, MA or 5-ml (Hamilton Company, Reno, NV) microsyringes. Microdialysates from both ears were collected alternately into two 10-$\mu$l or 25-$\mu$l sample loops of the 10-port valve body controlled by a sequence programmer (Valco Instruments Co. Inc., Houston, TX). A microdialysis perfusion flow rate of 0.5 $\mu$l/min was used in the study. The HPLC column was a YMC J'sphere® M80 4-$\mu$m reverse phase column (2 x 100 mm, 4 $\mu$m, Waters Corporation, Milford, MA). The mobile phase consisted of 0.1 % formic acid in water (pH = 2.8 - 2.9, 80% v/v) and acetonitrile, (20% v/v) with a flow rate of 0.1 ml/min. As the column performance changed with time, a different percent of acetonitrile (ranging from 19 to 25%) was used to optimize peak shape and retention times while maintaining the column temperature at 40°C. A Shimadzu 10-A HPLC system (Shimadzu Corporation, Kyoto, Japan) was employed to interface with the Valco on-line sample collection system. It consists of a LC-10AD*vp* pump, a SIL-10A system controller, a CTO-10A column heater, a FCV-10AL*vp* proportioner, and a DGU-14A degasser. The HPLC effluent entered the Turbo Ionspray source (400°C, 7 L/min nitrogen) of a PE-Sciex API-365 triple quadrupole MS-MS mass spectrometer (Perkin-Elmer Sciex Instruments, Concord, ON, Canada). The detection was conducted in multiple reaction monitoring (MRM) mode for the parent-product ion pair at 402.5 - 358.2 for moxifloxacin and 332 - 288 for the retrodialysis calibrator, ciprofloxacin. The concentration range applied for each experiment varied depending on the concentrations observed in the middle ear fluid dialysates. The lowest standard concentration used was 0.1 $\mu$g/ml while the highest was 118 $\mu$g/ml. As the standard concentration range was increased, there appeared to be slight nonlinearity with the signal increasing less than proportionally as concentration increased. To resolve this issue, a logarithmic transformation was applied to both the signal (peak area) and the standard concentration before performing linear regression with uniform weight.

*On Line HPLC-Fluorescence Analysis of Moxifloxacin and Ciprofloxacin in Microdialysates*

To improve productivity, an additional on line assay was developed to allow for multiple animal experiments to be conducted simultaneously, as well as to serve as a backup when equipment failure occurred. This assay involved the use of fluorescence detection at an excitation wavelength of 295 nm and an emission wavelength of 490 nm. A YMC ODS-A 5 $\mu$m, 120 A (4.6 x 100 mm, Waters Corporation, Milford, MA) column was employed with mobile phase composition of ammonium phosphate (20 mM, pH = 2.8, 76 or 78 %) and acetonitrile (24 or 22 %) and a flow rate of 0.5 ml/min. A column temperature of 45°C was employed to achieve retention times of 7.4 min for moxifloxacin and 3.6 min for ciprofloxacin. The fluorescence detector was either the Shimadzu RF 535 (Shimadzu, Kyoto, Japan) or the Jasco 821FP (Jasco Inc., Easton, MD). The other components and setup of the on-line system are identical to those outlined herein. The standard concentration range used for this assay was 0.1 to 205 $\mu$g/ml. Even though there did not appear to be any nonlinearity in the response signal for this assay, logarithmic transformation of the signal (peak area) and the standard concentration was conducted for the purpose of consistency before linear regression to obtain slope and $\gamma$-intercept.

*Precision and Accuracy in the Analysis of Moxifloxacin Middle Ear Fluid Microdialysis Samples By On Line HPLC-MS-MS and On Line HPLC-Fluorescence*

Precision and accuracy of both assay methods for moxifloxacin in microdialysates of middle ear fluid were evaluated from the standard curve readbacks. Six standards were typically used in the on-line HPLC-MS-MS assay, ranging in concentration from 0.5 to 100 (0.1 to 118) $\mu$g/ml. Accuracy was calculated to range from 96 to 105% over the range of 0.5 to 50 $\mu$g/ml. The accuracy at 100 $\mu$g/ml was 91 %, indicating a slight downward bias in the determination of microdialysate levels in this range. Precision ranged (%CV) from 1.1 to 6.2% over the entire range of the HPLC-MS-MS standard curve. Five or six standards were typically used in the on-line HPLC-fluorescence assay, ranging in concentration from 0.1 to 200 $\mu$g/ml. Accuracy was calculated to range from 98 to 102% over the entire range of the standard curve. Precision ranged (%CV) from 0.3 to 3.6% over the range of 0.5 to 200 $\mu$g/ml, and was 13.3% at 0.1 $\mu$g/ml.

*Assay for Total and Unbound Concentrations in Middle Ear Fluid and In Vitro Release Samples*

Moxifloxacin concentrations in protein binding study samples and *in-vitro* release samples were determined by HPLC (Shimadzu, Kyoto, Japan) with fluorescence detection. The components and chromatographic conditions were similar to those outlined herein. Processing of the *in vitro* release samples was as outlined herein. For the protein binding study samples, three separate standard curves were used; one for the ultrafiltrate and PBS samples and the other two for the determination of total drug concentration in AMEF and IMEF samples using their respective blank matrices. Samples having the highest concentration in spiked PBS, AMEF and IMEF were diluted three-fold with the corresponding blank matrices, and the ultrafiltrates from the highest concentration group were diluted two-fold with PBS before a 50-$\mu$l aliquot was taken for analysis. Internal standard (125 $\mu$l of 10 $\mu$g/ml ciprofloxacin) was added to all samples including the standard samples. To each sample, 200 $\mu$l of acetonitrile was added to precipitate the proteins. The samples were then vortexed and centrifuged at 2000 xg for 10 min before 100 $\mu$l of the supernatant was added to 300 $\mu$l of the filtered 20 mM ammonium phosphate monobasic so that the final sample resembled the mobile phase. An injection volume of 25 $\mu$l was used.

Example 9-Intrabulla Dosing Studies

These studies were performed to determine a unit impulse response function to be used in deconvolution analysis. To this end, the initial moxifloxacin concentration and half-life in the middle ear were estimated from intrabulla bolus dosing studies. All animals underwent obstruction of Eustachian tubes procedure as outlined herein prior to intrabulla dosing. Chinchillas were anesthetized with ketamine (40 to 50 mg/kg, IM) and pentobarbital (20 to 30 mg/kg, IP) during dose administration and implantation of microdialysis probe but were allowed to recover from anesthesia for the remainder of the experiment. Two dose levels were targeted. Bolus doses of 50 and 150 $\mu$g of moxifloxacin were delivered in 1 ml of AMEF directly into the middle ear (n = 9 ears) using the procedures as described herein. Unbound moxifloxacin concentrations from each ear (middle ear) were monitored every 20 min for up to 845 min post-dose using microdialysis coupled with HPLC-MS-MS or HPLC-fluorescence for a period of 4 to 5 half-lives.

Example 10-External Ear Gel Formulation Dosing Studies

Animals underwent bilateral Eustachian tube obstruction (ETO) surgery 1 day before dosing (except for 1 animal when ETO was performed on the day of dosing). Animals showing a negative pressure-reading from tympanometry indicated successful obstruction of Eustachian tubes. For the chinchilla (#638) that underwent ETO on the day of dosing, tympanometry was bypassed. The integrity of the tympanic membranes was checked visually using an otoscope following AMEF instillation, probe implantation, and before dosing. Ears showing compromised tympanic membranes were not dosed.

On the day of dosing, the animal was anesthetized with ketamine (40 to 50 mg/kg, IM) and pentobarbital (20 to 30 mg/kg, IP) and placed on a heating pad to maintain normal body temperature. Following tympanometry, the chinchilla was then placed on a mouth bar clamp to allow instillation of AMEF and probe implantation as outlined herein. Once the crowns were secured by dental cement, the animal was removed from the mouth clamp and placed on its side. The integrity of the tympanic membranes was confirmed with an otoscope once again.

Pre-treatment with a penetration enhancer in the form of 50 $\mu$l of 100% isopropyl myristate (IPM) was applied to the tympanic membrane using a piece of polyethylene (PE-50) tubing attached to a 1-ml tuberculin syringe. The PE-50 tubing was advanced into the external ear through the tip of the otoscope and positioned near the tympanic membrane with the aid of the otoscope. Once the isopropyl myristate was applied to the ear, a timer was activated and the PE-50 tubing was withdrawn. Four pretreatment times were included, 0.5 (immediately following isopropyl myristate instillation), 2, 5, and 20 min. Once the pretreatment time was reached, another piece of PE-tubing (PE-50 for the 1% moxigel and PE-160 for the 3% moxigel) attached to a 1-ml tuberculin syringe filled with the gel formulation was advanced into the external ear canal near the tympanic membrane with the aid of the otoscope. The gel formulation was applied slowly until it reached the otoscope tip. A timer was started, the PE-tubing and the otoscope were withdrawn while the external ear flap was pulled upward gently for at least 5 min to allow the formulation to gel. The external ear flap was released and a total gelation time of 10 min was allowed before dosing the contralateral ear. The animal's heart rate, respiration rate, body temperature, and depth of anesthesia were continually monitored throughout the procedures.

Once dosing of one or both ears was completed, an Elizabethan collar was fastened onto the animal before it was placed inside the Raturn® freely-moving animal caging system (Bioanalytical Systems, Inc., West Lafayette, IN) and allowed to recover fully from anesthesia. Middle ear fluid microdialysate samples were collected every 20 min from each ear and injected on-line for analysis in the HPLC-MS-MS system or in the HPLC-fluorescence system. Animals were allowed free access to food and water throughout the duration of the experiment. Buprenorphine (0.05 mg/kg IM) and subcutaneous fluid supplementation (6 mL of normal saline) was provided every 10 to 14 hours. Moxifloxacin concentrations in the middle ear fluid microdialysates were monitored for up to 5375 min following external ear dosing with 1 % moxigel and up to 6180 min following dosing with 3% moxigel.

Example 11-Data Analysis

*Correcting the Middle Ear Fluid Microdialysate Concentration for Probe Recovery and Lag Time*

Based on the length of the tubing connecting the microdialysis probe to the sample loop and the microdialysis perfusion flow rate, a lag time was estimated. This value represents the dialysate transit time from the tip of the probe to the sample collection loop. Typical lag times were about 45 min. The actual sample time was the mid-point of a 10-min collection interval, corrected for lag time and rounded to the nearest 5 min.

For each dialysate sample, the probe recovery was determined by subtracting the ratio of the calibrator peak area in the dialysate to that in the perfusate from unity. To reduce bias and to reflect the change in probe performance over the course of the experiment, a moving average of 5 estimates was used to correct for probe recovery instead of the "point-to-point" correction method. To obtain the actual middle ear fluid drug concentration, the moxifloxacin concentration in each dialysate was divided by the averaged probe recovery.

*Moxifloxacin Concentration-Time Data in Middle Ear Fluid Following Intrabulla Dosing*

Middle ear fluid levels of moxifloxacin in each of the nine (9) data sets were analyzed according to a monoexponential decline, where initial volume and elimination rate constant were estimated parameters. Nonlinear regression analysis (SAAM II, v 1.2.1, University of Washington, Seattle, WA) was utilized to characterize the parameters of a one-compartmental model. The weighting function assumed a coefficient of variation of 5%, and the variance model selected was associated with the data rather than the fitted function.

*Analysis of Moxifloxacin Concentrations in Middle Ear Fluid Following External Ear Dosing of 1% and 3% Moxifloxacin (Moxigel) Formulation*

Inspection of data involved a careful analysis of data sets where middle ear fluid concentrations were much higher than expected. Where confirmatory evidence suggested that these were the results from a compromised ear drum, the data set was excluded from further analysis. There were 23 usable data sets for the 1% moxigel applications, and 13 sets for the 3% moxigel applications. Moxifloxacin middle ear fluid concentration-time data were adjusted in time to the nearest 10 min in order to bin the data for subsequent noncompartmental analysis and deconvolution procedures. This "binning" step required an adjustment of no more than 10 min from the actual time to a nominal time.

Non-compartmental analysis of each middle ear fluid concentrations data set was performed with WinNonlin Professional (v 5.2, Pharsight Corporation, Mountain View, CA). Extravascular dosing (Model 200) was selected, with the linear trapezoidal and linear interpolation option. The "best fit for lambda_z" (the terminal rate constant) option, with log regression and uniform weighting, was selected. The extent of entry (bioavailability, %F) of moxifloxacin into the middle ear fluid was calculated for each data set from the external ear dose, the area under the curve from time 0 to infinity (AUCinf), and the mean elimination clearance from the middle ear fluid (CL) determined from the intrabulla dosing studies, as:

$$\%F = \frac{CL \cdot AUC\inf}{Dose} \qquad \text{eq. 1}$$

Deconvolution was performed within WinNonlin Professional (v 5.2, Pharsight Corporation, Mountain View, CA) to determine the input function (the rate of penetration of moxifloxacin into middle ear fluid) where the two-term unit impulse response function was defined using the mean volume of distribution and mean elimination rate constant determined from the analysis of the intrabulla dosing data. Unpaired t-tests showed that the volume and rate constant parameters were not significantly different at the two intrabulla dose levels.

Example 12-Release of Moxifloxacin From 1% and 3% Moxigel *In Vitro*

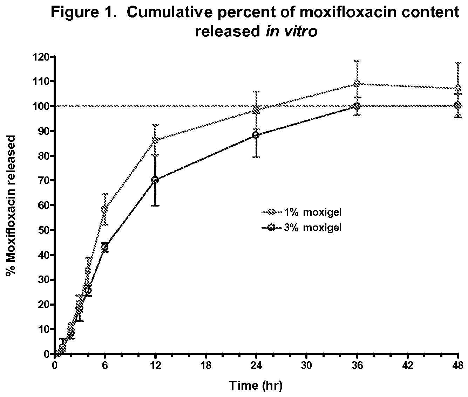

Plots of the cumulative percent of moxifloxacin released from the gel formulations, expressed with reference to the assayed concentration of moxifloxacin in the gel, are shown in Figure 1. After a modest initial release rate, both formulations exhibited maximum release rates *in vitro* occurring between about 2 to 6 hr. The release rates declined slowly thereafter.

Interpolation of the data revealed that the 1% moxigel released about 50% of its content in 5 hr, 75% in 9.5 hr, and 90% in 16 hr. The maximum release rate observed with the 1% moxigel formulation was approximately 12% per hr, corresponding to a release rate of about 9.4 $\mu$g/min from 500 $\mu$l of the formulation, a typical volume of gel dosed into the external ear with 1% moxigel.

The 3% moxigel released about 50% of its content in 7.5 hr, 75% in 15 hr, and 90% in 26 hr. The maximum release rate observed with the 3% moxigel formulation was approximately 9.1 % per hr, corresponding to a release rate of about 15 $\mu$g/min from 350 $\mu$l of the formulation, a typical volume of gel dosed into the external ear with 3% moxigel.

Although the 3% moxigel formulation exhibited higher absolute release rates of moxifloxacin than the 1% moxigel *in vitro*, the slower fractional release rates from the 3% moxigel is likely due to a portion of the dose being present in suspension, thus requiring some time for dissolution prior to release from the gel.

Example 13-Free Fraction of Moxifloxacin in Artificial and Incubated Middle Ear Fluid

The results from the protein binding study are summarized in Table 3. The free fractions observed for PBS were all close to 100% indicating little or no drug adsorption to the membrane of the ultrafiltration device. For AMEF, a very high percent unbound was calculated (84 to 92%) across the concentration range indicating minimal binding for moxifloxacin to bovine albumin. In IMEF, free fractions of moxifloxacin were lower than those observed in AMEF at similar concentrations suggesting the presence of another binding protein in this matrix.

Overall, the free fraction for moxifloxacin remained relatively high in the concentration range tested. Thus, the drug concentrations measured by microdialysis represent a large portion of the total moxifloxacin level present in the middle ear fluid. During the first few hours following dosing, the sample matrix closely resembles AMEF. As the experimental time approaches 18 or more hours, it is anticipated that the sample matrix is better represented by IMEF. From the results presented in Table 3, there was a significant difference between free fractions in the two matrices. However, this difference is not likely to have any meaningful impact on the interpretation of the MEF microdialysis data.

Table 3. Percent Unbound of Moxifloxacin in Incubated Middle Ear Fluid (IMEF), Artificial Middle Ear Fluid (AMEF), and Phosphate Buffered Saline (PBS)

| Spiked sample matrix | Nominal spiked concentration ($\mu$g/ml) | Averaged measured concentration ($\mu$g/ml) | Percent unbound (%) (mean $\pm$ SD) |
|---|---|---|---|
| IMEF | 220 | 175 | 68 $\pm$ 0.4 |
| | 492 | 387 | 75 $\pm$ 2.0 |
| AMEF | 220 | 182 | 84 $\pm$ 6.3 |
| | 492 | 369 | 92 $\pm$ 1.3 |
| PBS | 220 | 200 | 97 $\pm$ 2.6 |
| | 492 | 421 | 105 $\pm$ 6.0 |

Example 14-Middle Ear Fluid Concentrations of Moxifloxacin Following Intrabulla Dosing

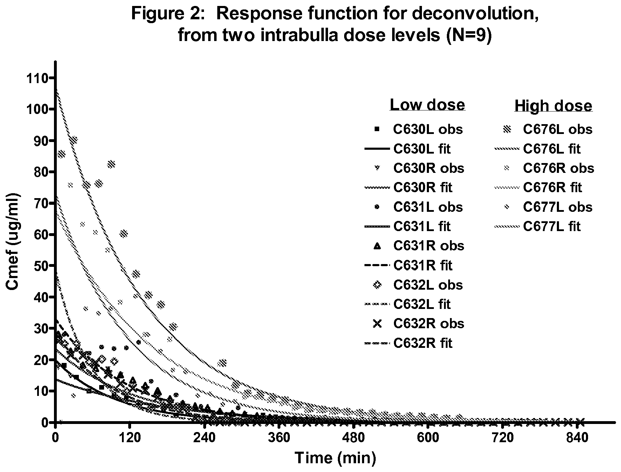

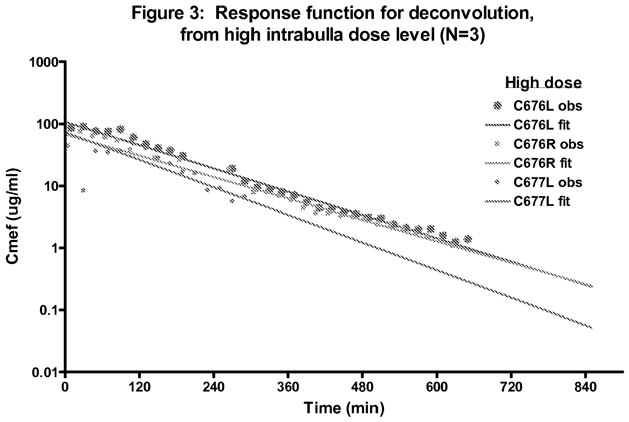

Plots of the unbound middle ear fluid moxifloxacin concentrations (Cmef) determined by microdialysis, following dosing directly into the middle ear at approximately 50 and 150 $\mu$g are shown in Figures 2 and 3. The middle ear fluid moxifloxacin concentrations declined monoexponentially, exhibiting relatively modest intersubject variability. These concentration-time profiles were used to define the unit impulse response function (UIRF) in the deconvolution analysis of the Cmef data following trans-tympanic membrane delivery. The volumes of distribution of moxifloxacin in middle ear fluid were estimated to be 1.75 ± 0.79 and 1.90 ± 0.43 ml (mean ± SD) for the low and high intrabulla doses, respectively. The elimination rate constants were estimated to be 0.0102 ± 0.0040 and 0.0075 ± 0.0010 min$^{-1}$, respectively. Neither the volume nor the elimination rate constant was dose dependent. Thus, the mean values for these two parameters were used to define the UIRF in the deconvolution procedure.

Example 15-Middle Ear Fluid Concentrations of Moxifloxacin Following External Ear Dosing with 1% and 3% Moxigel

*External Ear Dosing with 1% Moxigel*

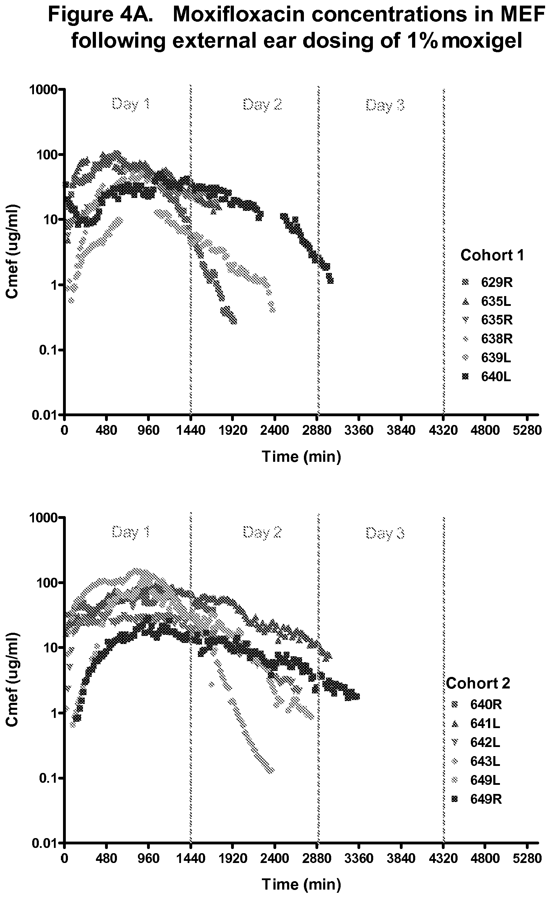

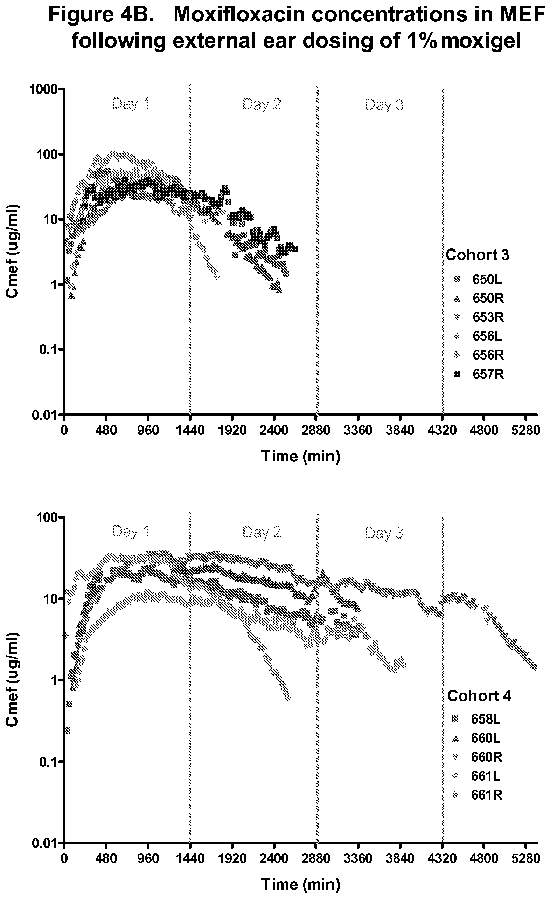

Plots of the middle ear fluid moxifloxacin concentrations (Cmef) following external ear dosing with 1% moxigel are shown in Figures 4A and 4B, in groups. Measurable levels were obtained for up to 4 days (5375 min) after dosing. The length of the pretreatment time with IPM (0.5, 2, 5 or 20 min) had no effect on the Cmax or AUCinf values determined in these studies.

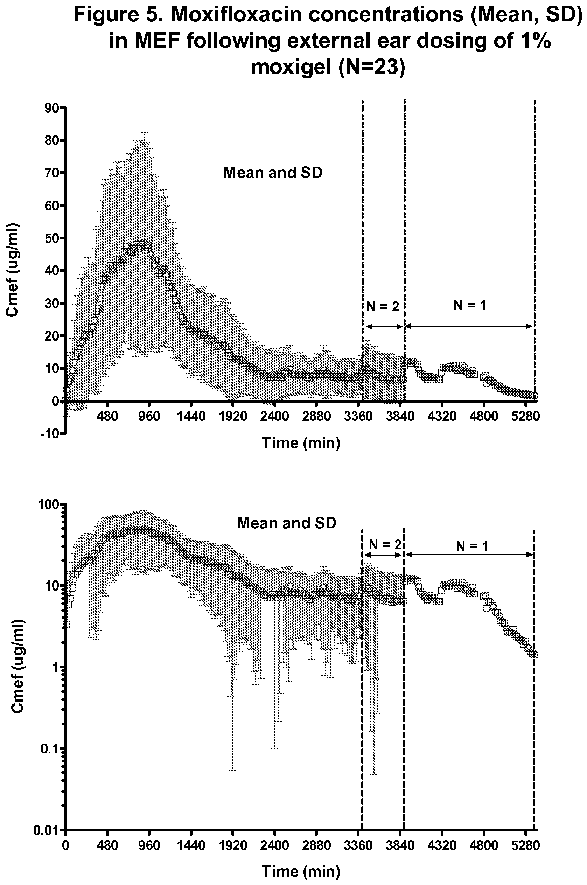

These data are presented as mean ± SD on linear and log concentration scales in Figure 5. Maximum mean concentrations of moxifloxacin were approximately 48 μg/ml, and occurred at about 900 min (15 hr) post-dose. Mean concentrations of 10 μg/ml were reached at about 90 min and remained above that level until about 2100 min (35 hr) following dosing. Mean concentrations of 20 μg/ml were reached at about 230 min and remained above that level until about 1610 min (27 hr) following dosing.

Results of noncompartmental analysis of the unbound (free) middle ear fluid concentration-time data are summarized in Table 4. Significant variability was observed in Cmax and the terminal rate constant, $\lambda_z$. The latter parameter describes the fractional rate of decline of Cmef, and reflects the rate-limiting step in the entry and exit of moxifloxacin into/from the middle ear. Here, the rate-limiting step is the rate of penetration of the antibiotic across the tympanic membrane. This is evident because the mean rate constant associated with elimination from middle ear fluid, as determined in the intrabulla dosing study, was in the range of 0.008 to 0.010 min$^{-1}$, substantially greater than the mean value of 0.0043 determined here.

Table 4. Moxifloxacin Parameters from NCA of Cmef Data Following External Ear Dosing with 1% Moxigel (N=23)

| | $\lambda_z$ | half-life | Tmax | Cmax | AUCinf | %F |
|---|---|---|---|---|---|---|
| | 1/min | (min) | (min) | (μg/mL) | min*μg/mL | |
| mean | 0.00403 | 381 | 870 | 57.8 | 65712 | 23.7 |
| SD | 0.00424 | 323 | 239 | 36.9 | 34760 | 12.6 |
| %CV | 105.4 | 84.9 | 27.5 | 63.7 | 52.9 | 53.2 |
| min | 0.001 | 41 | 400 | 12.1 | 12889 | 3.8 |
| max | 0.017 | 1101 | 1220 | 153.1 | 143175 | 46.2 |

The mean maximum value of unbound Cmef, 57.8 μg/ml, is 20 to 30 times higher than that observed in plasma (2.0 μg/ml) following an oral dose of 400 mg in humans (Owens & Ambrose, 2002, Pharmacodynamics of Quinolones, In "Antimicrobial Pharmacodynamics in Theory and Clinical Practice, pg 162, Eds, Nightingale, Marakawa and Ambrose, Marcel Dekker, Basel, CH). The bioavailability (%F) of moxifloxacin from 1% moxigel into the middle ear fluid was calculated as described in eq. 1.

Table 5A summarizes the time required for Cmef to reach, fall below, and the duration above, 10 μg/ml. The tabulated values were obtained by inspection of each data set following dosing with 1% moxigel (N=23). The average time that Cmef levels remained above 10 μg/ml was over 1700 min (approximately 29 hr).

Table 5A. Time to Reach, Fall Below, and Time Over 10 μg/ml Following External Ear Dosing with 1% Moxigel

| | time to reach 10 μg/ml | time to fall below 10 μg/ml | time over 10 μg/ml |
|---|---|---|---|
| | (min) | (min) | (min) |
| Mean | 223 | 1971 | 1749 |
| SD | 202 | 675 | 719 |
| %CV | 90.8 | 34.2 | 41.1 |
| Median | 180 | 1780 | 1660 |
| Min | 20 | 1200 | 460 |
| Max | 800 | 4060 | 3720 |

Table 5B summarizes the time required for Cmef to reach, fall below, and the duration above 20 μg/ml. The tabulated values were obtained by inspection of each data set following dosing with 1% moxigel (N=23). The average time that Cmef levels remained above 20 μg/ml was over 1100 min (approximately 19 hr). Cmef in two of the data sets did not rise to 20 μg/ml, resulting in a value of 0 min for 2 data sets, for "time over 20 μg/ml".

Table 5B. Time to Reach, Fall Below, and Time Over 20 μg/ml Following External Ear Dosing with 1% Moxigel

|  | time to reach 20 μg/ml (min) | time to fall below 20 μg/ml (min) | time over 20 μg/ml (min) |
|---|---|---|---|
| Mean | 326 | 1569 | 1135 |
| SD | 218 | 407 | 573 |
| %CV | 67.0 | 26.0 | 50.5 |
| Median | 300 | 1520 | 1160 |
| Min | 60 | 1020 | 0 |
| Max | 860 | 2620 | 2340 |

*External Ear Dosing with 3% Moxigel*

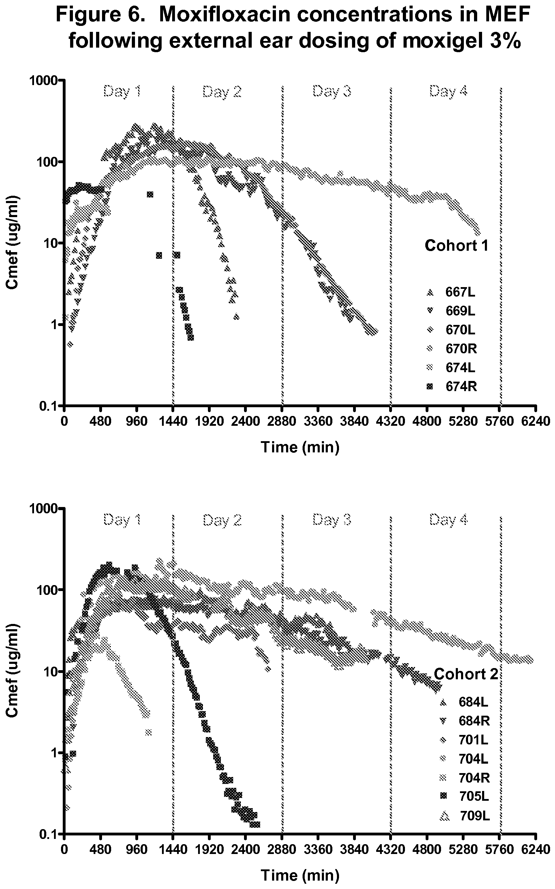

Plots of the middle ear fluid moxifloxacin concentrations (Cmef) following external ear dosing with 3% moxigel are shown in Figure 6, by group. Measurable levels were obtained for up to 4 days (6180 min) after dosing. As was the case with the 1% moxigel dosing data, the length of the pretreatment time with IPM had no effect on the Cmax or AUCinf determined on these studies.

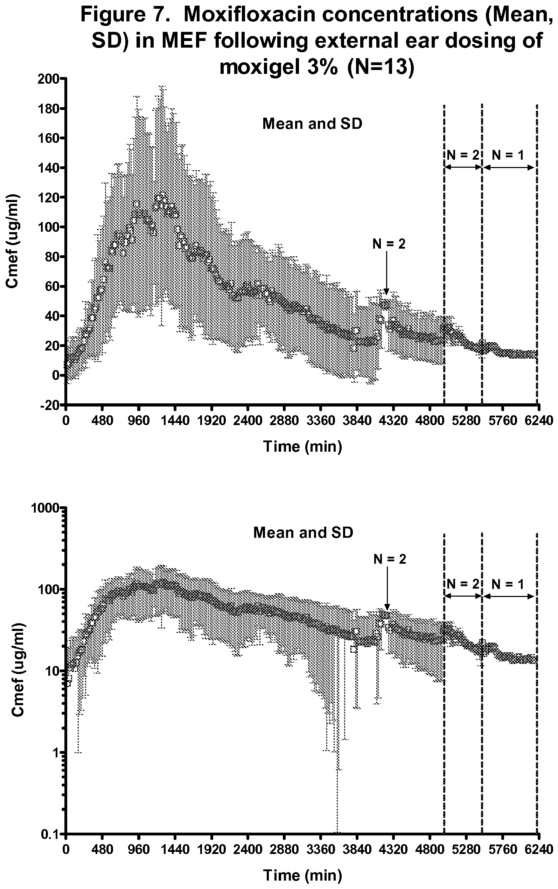

These data are presented as mean ± SD on linear and log concentration scales in Figure 7. Maximum mean concentrations of moxifloxacin were approximately 120 μg/ml, and occurred at about 1300 min (22 hr) post-dose. Mean concentrations of 10 μg/ml were reached at about 60 min and remained above that level throughout the study period (over 5000 min) following dosing. Mean concentrations of 20 μg/ml were reached at about 230 min and remained above that level until about 5340 min (89 hr). Because there were only 2 data sets that exhibited measureable levels beyond 57 hr, this observation does not reflect the overall trend of the data.

Results of noncompartmental analysis of the unbound middle ear fluid concentration-time data following external ear dosing of 3% moxigel are summarized in Table 6.

Table 6. Moxifloxacin Parameters from NCA of Cmef Data Following External Ear Dosing with 3% Moxigel (N=13)

|  | $\lambda_z$ 1/min | half-life (min) | Tmax (min) | Cmax (μg/μL) | AUCinf min*μg/mL | %F |
|---|---|---|---|---|---|---|
| mean | 0.00288 | 493 | 1049 | 130.4 | 206398 | 34.8 |
| SD | 0.00203 | 470 | 534 | 79.5 | 130684 | 21.7 |
| %CV | 70.5 | 95.4 | 50.9 | 61.0 | 63.3 | 62.4 |
| min | 0.00040 | 116 | 200 | 27.2 | 12311 | 2.9 |
| max | 0.00600 | 1623 | 1900 | 273.6 | 493290 | 77.5 |

As with 1% moxigel, significant variability was observed in Cmax and the terminal rate constant, $\lambda_z$. The mean value of this rate constant was 0.0029 min$^{-1}$, substantially less than the elimination rate constant determined following intrabulla dosing. This corresponds to a geometric mean half-life of about 350 min, much longer than the elimination half-life observed when moxifloxacin is dosed directly into the middle ear fluid. This is evidence that, as in the case of the 1% moxigel studies, penetration of moxifloxacin across the tympanic membrane is limiting the rate of disappearance from middle ear fluid. The mean bioavailability of the external ear dose into middle ear fluid was approximately 35%, but was quite variable. This was likely due to variability in contact of the gel with the tympanic membrane, perhaps resulting in part from inadequate contact between the gel and the membrane. In spite of this variability in %F, very high maximum middle ear fluid concentrations of moxifloxacin were observed in this study, averaging 130 μg/ml. Indeed, the data set that showed a bioavailability of 2.9% exhibited a Cmax of 27.7 μg/ml, more than 10 times the maximum unbound plasma concentration usually observed following an oral dose of 400 mg of moxifloxacin (Owens et al., *supra*).

Table 7A summarizes the time required for Cmef to reach, fall below, and the duration above, 10 μg/ml. The tabulated values were obtained by inspection of each data set following dosing with 3% moxigel (N=13). The average time that Cmef levels remained above 10 μg/ml was over 2800 min (approximately 48 hr).

Table 7A. Time to Reach, Fall Below, and Time Over 10 µg/ml Following External Ear Dosing with 3% Moxigel

|  | time to reach 10 ug/ml (min) | time to fall below 10 ug/ml (min) | time over 10 ug/ml (min) |
|---|---|---|---|
| Mean | 172 | 3020 | 2848 |
| SD | 107 | 1762 | 1750 |
| %CV | 62 | 58 | 61 |
| Median | 180 | 3140 | 2740 |
| Min | 20 | 580 | 500 |
| Max | 400 | 6180 | 5960 |

Table 7B summarizes the time required for Cmef to reach, fall below, and the duration above 20 µg/ml. The tabulated values were obtained by inspection of each data set following dosing with 3% moxigel (N=13). The average time that Cmef levels remained above 20 µg/ml was over 2500 min (approximately 42 hr).

Table 7B. Time to Reach, Fall Below, and Time Over 20 µg/ml Following External Ear Dosing with 3% Moxigel

|  | time to reach 20 ug/ml min | time to fall below 20 ug/ml min | time over 20 ug/ml min |
|---|---|---|---|
| mean | 237 | 2769 | 2532 |
| SD | 127 | 1600 | 1583 |
| %CV | 53 | 58 | 63 |
| median | 240 | 2920 | 2420 |
| min | 20 | 580 | 220 |
| max | 500 | 5520 | 5300 |

Example 16-Comparative Metrics for Moxifloxacin Following External Ear Dosing with 1% and 3% Moxigel

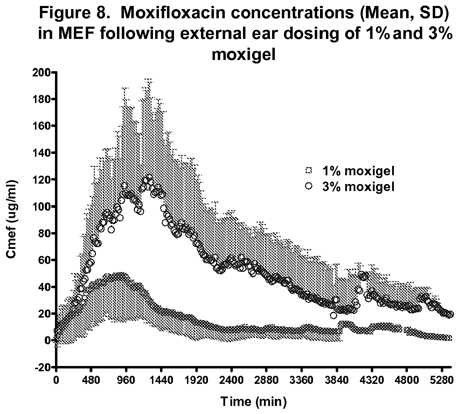

A comparison of the middle ear fluid concentration (Cmef)-time profiles following external ear dosing with 1% and 3% moxigel is provided in Figure 8, which plots means and SD of the middle ear fluid moxifloxacin concentrations against time. Because the volumes of the respective formulations introduced into the external ear were not the same (averaging approximately 500 and 350 µl for 1% moxigel and 3% moxigel, respectively), the doses placed in the external ear were, on average, about two-fold greater in the 3% moxigel cohorts than in the 1% moxigel group (9900 compared with 4800 µg). In addition, the time course of penetration was significantly prolonged in the 3% moxigel cohort, likely as a result of its slower moxifloxacin relative release rates (see Figure 1). These differences in dose and release rates are evident in Figure 8, which demonstrates higher average Cmax values for 3% moxigel and the apparently later times at which the maximum of the mean Cmef values was reached.

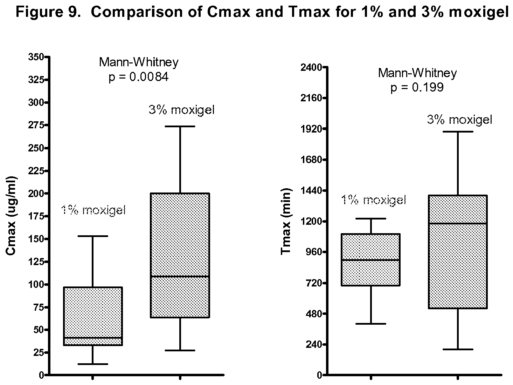

A comparison of the Cmax and Tmax values for middle ear fluid moxifloxacin levels observed for these two formulations is shown in the comparative box and whisker plots in Figure 9. These plots show the median and interquartile range for these metrics. These plots also indicate that, although the median Tmax values for the two formulations (900 vs. 1180 min for 1% moxigel and 3% moxigel, respectively) were not different by the nonparametric Mann-Whitney test, the median Cmax values differed significantly (41.2 vs. 109 µg/ml for 1% moxigel and 3% moxigel, respectively) with a p-value of 0.0084.

Example 17-Rate and Extent of Penetration of Moxifloxacin into Middle Ear Fluid

Using the results of the intrabulla dosing studies and the time course of Cmef following external ear dosing with moxigel, deconvolution was used to calculate the rate and extent of trans-tympanic membrane penetration of moxifloxacin.

*Penetration of Moxifloxacin into Middle Ear Fluid using 1% Moxigel*

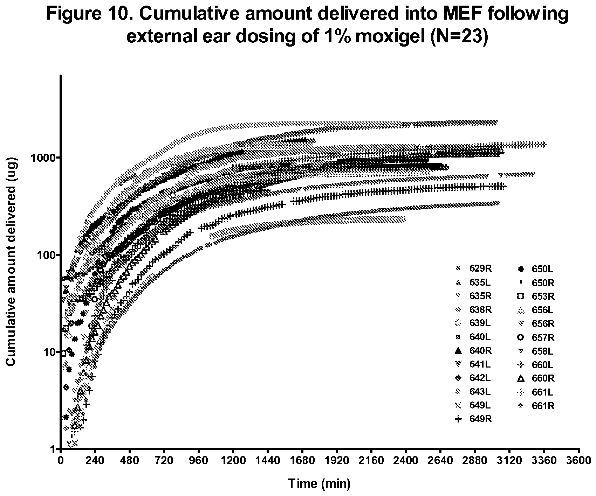

The time course of the cumulative amount of moxifloxacin reaching the middle ear fluid following external ear dosing with 1% moxigel is shown in Figure 10. The cumulative amounts delivered to middle ear fluid ranged from about 250 to 2300 µg, and this range is reflective of the range of AUCinf values observed for dosing with 1% moxigel.

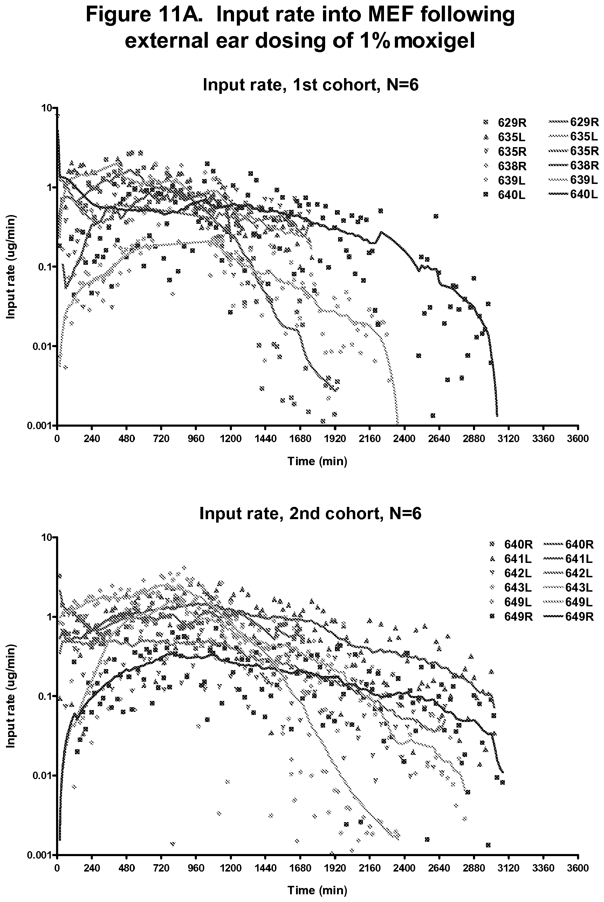

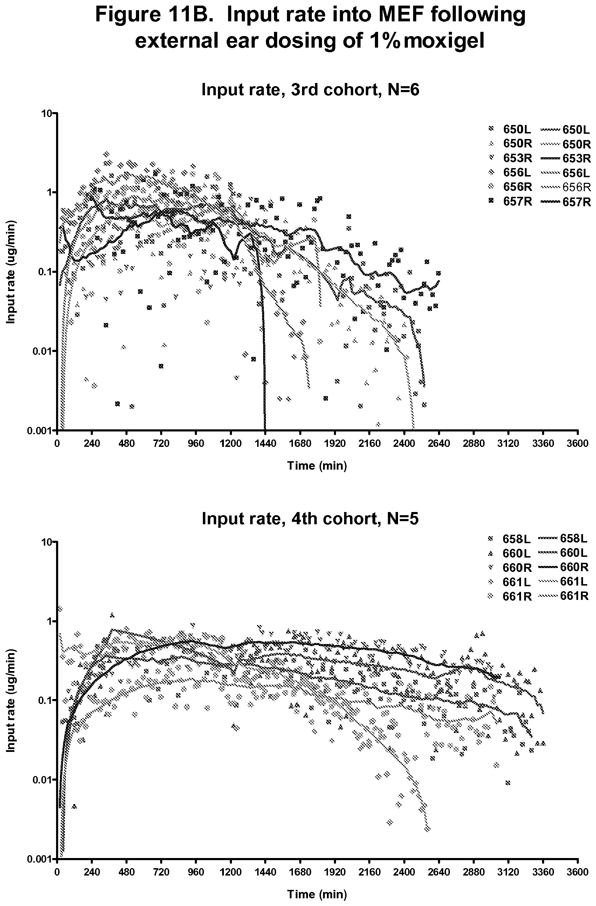

The corresponding rates of penetration (input rates), also calculated by deconvolution, are presented graphically, by group, in Figures 11A and 11B. A spline function was fitted to each cohort to reflect the overall trend of input rate in that group of data sets. This was done using locally weighted scatterplot smoothing, employing a span of 10 data points in the procedure. Inspection of the splines in Figures 11A and 11B indicates that the maximum penetration rates of moxifloxacin into the middle ear fluid ranges from about 0.2 to 2 $\mu$g/min, significantly less than the corresponding release rate estimated for a comparable dose of 1% moxigel under *in vitro* conditions (9.4 $\mu$g/min). These maximum penetration rates roughly correspond to moxifloxacin delivery rates *in vivo* of about 12 to 120 $\mu$g/hr.

*Penetration of Moxifloxacin into Middle Ear Fluid using 3% Moxigel*

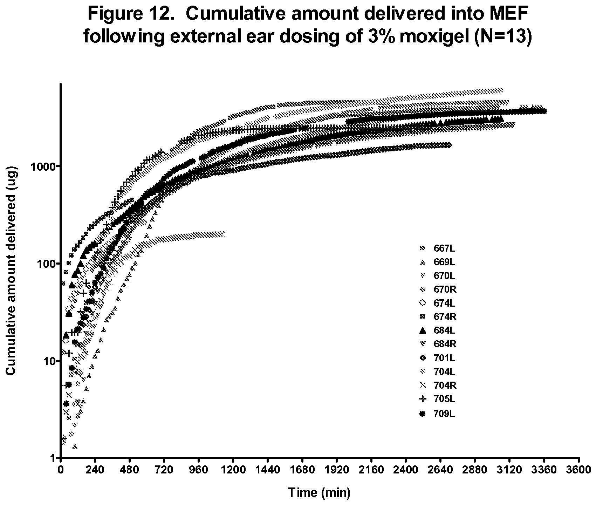

The time course of the cumulative amount of moxifloxacin reaching the middle ear fluid following external ear dosing with 3% moxigel is shown in Figure 12. The cumulative amounts delivered to middle ear fluid ranged from about 200 to 6000 $\mu$g, and this approximately reflects the range of AUCinf values observed for dosing with 3% moxigel.

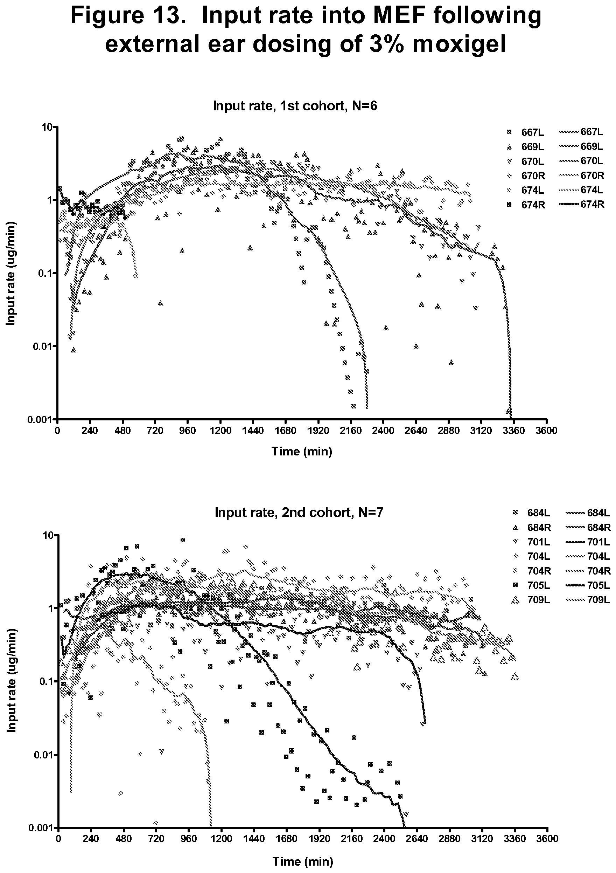

The corresponding rates of penetration (input rates) calculated by deconvolution are presented graphically, by group, in Figure 13. Spline functions fitted to each cohort reflecting the overall trend of input rate in those data are also shown. Inspection of the splines in Figure 13 indicates that the maximum penetration rates of moxifloxacin into the middle ear fluid range from about 0.5 to 5 $\mu$g/min, significantly less than the corresponding release rate estimated for a comparable dose of 3% moxigel under *in vitro* conditions (15 $\mu$g/min). These maximum penetration rates roughly correspond to moxifloxacin delivery rates *in vivo* of about 30 to 300 $\mu$g/hr.

*Comparative Penetration Rates of Moxifloxacin into Middle Ear Fluid for 1% Moxigel and 3% Moxigel.*

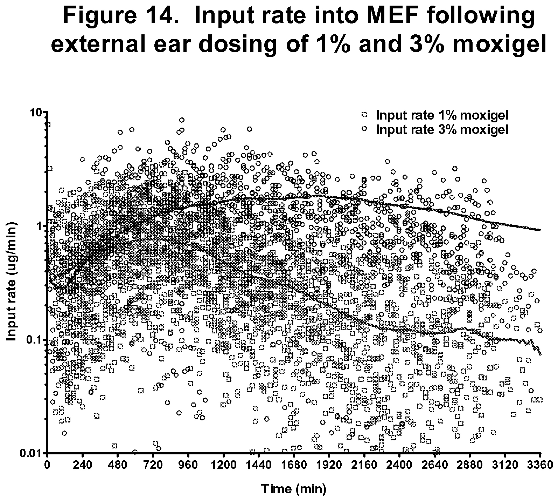

Rates of penetration (input rates) estimated by deconvolution for each of the 1% moxigel and 3% moxigel data sets (N=36) were fit to data, grouped by dose, by spline functions as described above. The data and the corresponding input rate splines are presented graphically in Figure 14. These plots show that the typical maximum penetration (input) rate for 1% moxigel is approximately 0.7 $\mu$g/ml, and occurs at about 700 to 800 min (approximately 12 to 13 hr). The corresponding maximum penetration rate for 3% moxigel is about 2 $\mu$g/min, and occurs in the range of 1600 to 2000 min (approximately 27 to 33 hr). The higher maximum input rate seen with 3% moxigel is consistent with the higher dose associated with this formulation. It should be noted that the typical external ear dose using 1% moxigel was about 4800 $\mu$g, and the corresponding dose using 3% moxigel, which was typically administered in a smaller volume, was about 9900 $\mu$g. Thus, the dose associated with 3% moxigel was about twice that for 1% moxigel. The later peak penetration rate observed with 3% moxigel reflects the fact that moxifloxacin is released more slowly from this formulation *in vitro*, and presumably *in vivo,* since it is partially in suspension in the 3% gel formulation.

Example 18-Time to Reach, and Duration Above, Target Moxifloxacin Concentrations in Middle Ear Fluid

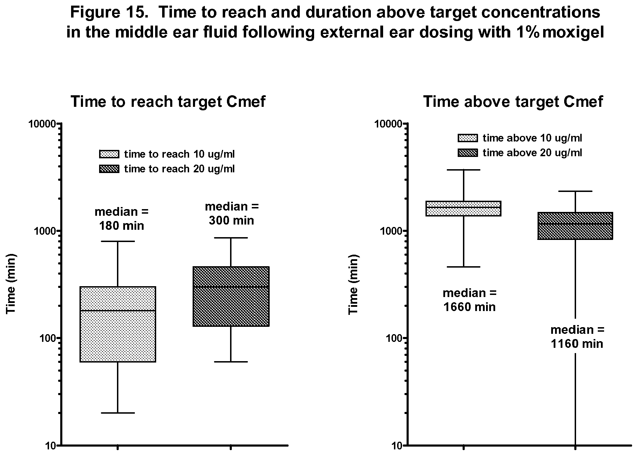

A specific aim of this research was to develop a trans-tympanic membrane delivery system to maintain moxifloxacin concentrations in the middle ear fluid above 10 $\mu$g/ml for a minimum of 24 to 48 hr. Figure 15 shows the median and interquartile ranges for the time required to reach both 10 and 20 $\mu$g/ml seen in the studies involving external ear dosing with 1% moxigel. The median times required to achieve middle ear fluid levels of 10 and 20 $\mu$g/ml were 180 and 300 min, respectively. The figure also shows the median time during which these levels were maintained. These were 1660 and 1160 min, or approximately 28 and 19 hr, respectively. Thus, this aim appears to have been met with the 1% moxigel formulation.

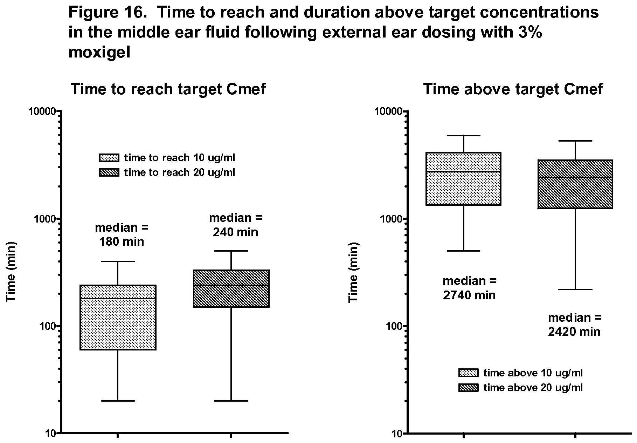

In addition, Figure 16 shows the median and interquartile ranges for the time required to reach both 10 and 20 $\mu$g/ml seen in the studies with 3% moxigel. The median times required to achieve middle ear fluid levels of 10 and 20 $\mu$g/ml were 180 and 240 min, respectively. The figure also shows the median time during which these levels were maintained. These were 2740 and 2420 min, or approximately 46 and 40 hr, respectively. The aim described above, which relates to duration over 10 $\mu$g/ml, has also been achieved with the 3% moxigel formulation. In addition, although this was not explicitly stated as an aim, the 3% moxigel formulation provided a median duration of time over 20 $\mu$g/ml of almost two days. That this formulation appeared to include moxifloxacin in suspension as well as in solution probably afforded a prolonged release rate *in vivo,* in accord with its slower relative rate of release *in vitro.*

The times and durations shown in Figures 15 and 16 are summarized in Tables 8A and 8B as well as in Tables 9A and 9B for 1% moxigel and 3% moxigel, respectively. Mean times, as well as minimum and maximum times observed in individual data sets are also tabulated.

Table 8A. Time to Reach, Fall Below, and Time Over 10 $\mu$g/ml Following External Ear Dosing with 1% Moxigel

|  | time to reach 10 $\mu$g/ml min | time to fall below 10 $\mu$g/ml min | time over 10 $\mu$g/ml min |
|---|---|---|---|
| mean | 223 | 1971 | 1749 |
| SD | 202 | 675 | 719 |
| %CV | 90.8 | 34.2 | 41.1 |
| median | 180 | 1780 | 1660 |
| min | 20 | 1200 | 460 |
| max | 800 | 4060 | 3720 |

Table 8B. Time to Reach, Fall Below, and Time Over 20 $\mu$g/ml Following External Ear Dosing with 1% Moxigel

|  | time to reach 20 ug/ml min | time to fall below 20 ug/ml min | time over 20 ug/ml min |
|---|---|---|---|
| mean | 326 | 1569 | 1135 |
| SD | 218 | 407 | 573 |
| %CV | 67.0 | 26.0 | 50.5 |
| median | 300 | 1520 | 1160 |
| min | 60 | 1020 | 0 |
| max | 860 | 2620 | 2340 |

Table 9A. Time to Reach, Fall Below, and Time Over 10 $\mu$g/ml Following External Ear Dosing with 3% Moxigel

|  | time to reach 10 ug/ml min | time to fall below 10 ug/ml min | time over 10 ug/ml min |
|---|---|---|---|
| mean | 172 | 3020 | 2848 |
| SD | 107 | 1762 | 1750 |
| %CV | 62 | 58 | 61 |
| median | 180 | 3140 | 2740 |
| min | 20 | 580 | 500 |
| max | 400 | 6180 | 5960 |

Table 9B. Time to Reach, Fall Below, and Time Over 20 $\mu$g/ml Following External Ear Dosing with 3% Moxigel

|  | time to reach 20 ug/ml min | time to fall below 20 ug/ml min | time over 20 ug/ml min |
|---|---|---|---|
| mean | 237 | 2769 | 2532 |
| SD | 127 | 1600 | 1583 |
| %CV | 53 | 58 | 63 |
| median | 240 | 2920 | 2420 |
| min | 20 | 580 | 220 |
| max | 500 | 5520 | 5300 |

Example 19-AUIC Ratios for Moxifloxacin Following 1% and 3% Moxigel External Ear Dosing

Three important break points have been proposed for fluoroquinolones, as linked to bacterial killing rates by Schentag et al. (2003, Ann. Pharmacother., 37:1287-98). These break points have been established *in vitro* and in animal models in view of the apparent concentration-dependent killing of this class of antibiotics. AUIC is defined as the area-under-the-curve (plasma or serum concentrations) over 24 hr, divided by the minimum inhibitory concentration of the fluoroquinolone in question for a particular organism. Schentag et al. (*supra*) have reported the following break points for fluoroquinolones:

a. at AUIC values < 30-50 or peak:MIC ratios in the range of 5:1, fluoroquinolones are bacteriostatic
b. at AUIC values > 100 but < 250 organisms are killed at a slower rate, usually by day 7 of treatment
c. at AUIC > 250 or peak:MIC of 25:1, fluoroquinolones demonstrate rapid concentration-dependent killing, and bacterial eradication occurs within 24 hours.

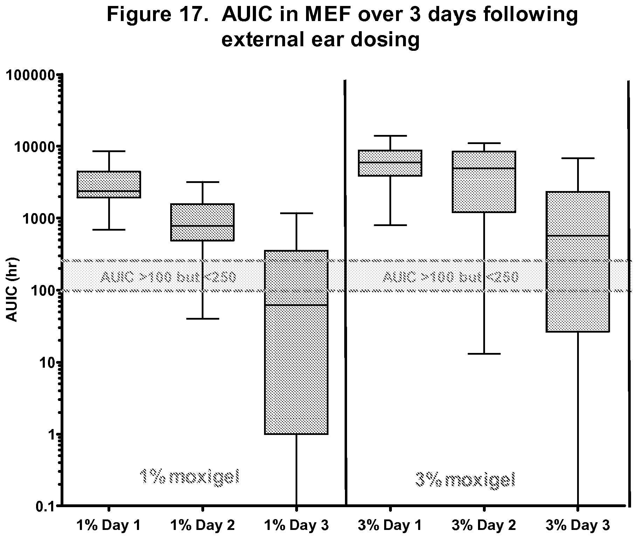

AUIC values for all data sets in the 1% moxigel and 3% moxigel studies were calculated for each consecutive 24-hr period following external ear dosing. These calculations assumed an MIC for moxifloxacin of 0.25 $\mu$g/ml. The median AUIC values in middle ear fluid following 1% moxigel dosing (N=23) were 2398, 7756, and 62 on days 1, 2, and 3, respectively, as shown in Figure 17. These values for 1% moxigel fall within category c, identified above, for Day 1 and Day 2, i.e., eradication of bacteria within 24 hr. The median AUIC determined on Day 3 for the 1% moxigel studies lies between categories b and a.

Median AUIC values in middle ear fluid following 3% moxigel dosing (N=13) were 5930, 4901, and 570 on days 1, 2, and 3, respectively, also shown in Figure 17. These values for 3% moxigel fall within category c, identified above, for all 3 days following external ear dosing, i.e., eradication of bacteria within 24 hr. This finding suggests that the time-course of concentrations of moxifloxacin in the middle ear fluid of the chinchilla following 3% moxigel would provide a real advantage over those observed with the application of 1% moxigel. However, if Schentag et al. are correct in their recommendations, the levels of moxifloxacin produced by external ear dosing with 1% moxigel may be adequate, since the associated AUICs on Day 1 and Day 2 appear to be sufficient to result in bacterial eradication within 24 hours.

An MIC for moxifloxacin of 0.25 $\mu$g/ml in the AUIC calculations above is reasonable, and perhaps conservative. In a subsequent paper, Schentag et al. (2003, Ann. Pharmacotherap., 37:1478-88) focus on the use of break points for the assessment of bactericidal effects of the fluoroquinolones, and report an MIC90 of 0.125 $\mu$g/ml for moxifloxacin against strains of S. *pneumoniae,* a common pathogen encountered in otitis media. Of interest, this publication examines the outcomes of human clinical trials and confirms the break points previously reported. Indeed, the authors state that the concentration-dependent bacterial killing by fluoroquinolones results in bacterial eradication in 1 to 2 hrs in humans, where the AUIC is greater than 250, or Cmax to MIC ratios are in excess of 15:1.

These published break points do not appear to consider protein binding. The measured middle ear fluid concentrations in the current studies are unbound levels, and, if total levels were used in calculating the AUICs produced, these would be somewhat higher, placing them more firmly into class c-where fluoroquinolones demonstrate rapid concentration-dependent killing and bacterial eradication occurs within 24 hours.

Example 20-Cmax/MIC Ratios for Moxifloxacin Following 1% Moxigel and 3% Moxigel External Ear Dosing

The break points discussed in Example 19 above also included criteria related to Cmax/MIC ("peak:MIC") ratios. Focusing on these ratios in classes a and c from above, Schentag et al. (2003, Ann. Pharmacother., 37:1287-98) stated that:

a. at peak:MIC ratios in the range of 5:1, fluoroquinolones are bacteriostatic.
c. at peak:MIC of 25:1, fluoroquinolones demonstrate rapid concentration-dependent killing, and bacterial eradication occurs within 24 hours.

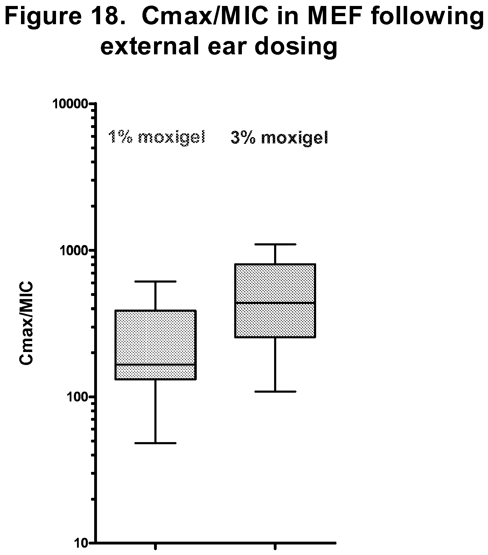

The break points were revised slightly in the second publication (Schentag et al., 2003, Ann. Pharmacotherap., 37:1478-88), reporting Cmax/MIC ratios for class a, b, and c to be 3:1, 6:1, and 15:1, respectively. Cmax/MIC for all data sets in the 1% moxigel and 3% moxigel studies were calculated for each consecutive 24-hr period following external ear dosing. As before, these calculations assumed an MIC for moxifloxacin of 0.25 $\mu$g/ml. The median Cmax/MIC value in middle ear fluid following 1% moxigel dosing (N=23) was 165:1, as shown in Figure 18. This value is several times that identified for class c (alternately reported as 25:1 or 15:1) where eradication of bacteria within 24 hr is expected.

The median Cmax/MIC value in middle ear fluid following 3% moxigel dosing (N=13) was 436:1, as shown in Figure 18. This value is many-fold higher than that identified for class c, again suggesting eradication of bacteria within 24 hr.

As noted above, the published break points do not consider protein binding. The measured middle ear fluid concentrations in the current studies are unbound levels, and, if total levels were used in calculating the Cmax/MIC ratios considered in the reported break points, these ratios would be somewhat higher, placing them even more firmly into class c, where fluoroquinolones demonstrate rapid concentration-dependent killing with bacterial eradication occurring within 24 hours.

Example 21-Methods to Compare Pre-Treatment with a 10% vs. 50% Penetration Enhancer

Animals underwent bilateral Eustachian tube obstruction (ETO) surgery 1 day before dosing. Ears showing a negative pressure reading from tympanometry indicated successful obstruction of Eustachian tubes. The integrity of the tympanic membranes was checked visually using an otoscope following artificial middle ear fluid (AMEF) instillation into the bulla, probe implantation, and before dosing. Ears showing compromised tympanic membranes were not dosed.

On the day of dosing, the animal was anesthetized with ketamine (40 to 50 mg/kg, IM) and pentobarbital (20 to 30 mg/kg, IP) and placed on a heating pad to maintain normal body temperature. Following tympanometry, the chinchilla was placed on a mouth bar/clamp to allow instillation of AMEF and microdialysis probe implantation. Access to the chinchilla middle ear cavity was through the cephalad bulla on the dorsal side of the skull. A small hole was drilled manually with a 15 GA hypodermic needle at the apex of the right and left bulla where the bone is thin. AMEF was instilled into each bulla via a length of PE-50 tubing until it was completely filled to the top.

Implantation of microdialysis probes (MD-2310) with 10 mm membranes (BASi, West Lafayette, IN) in both the left and right middle ear bullas immediately followed AMEF instillation. Access to the chinchilla middle ear cavity was through the same hole on the cephalad bulla. A probe was carefully inserted into each middle ear cavity through the access hole. The integrity of the tympanic membrane was examined using an otoscope. The probes were secured onto the chinchilla skull using a plastic crown secured by dental cement and anchor needles.

Prior to dosing with a 3% moxigel solution into the external ear as described above, pretreatment with a penetration enhancer, in the form of either a 10% or 50% v/v solution of isopropyl myristate (IPM) in mineral oil, was performed. This consisted of applying 50 $\mu$L of the pretreatment solution into the region of the tympanic membrane via the external ear with the aid of an otoscope. This was accomplished using a piece of polyethylene (PE-50) tubing attached to a 1-mL tuberculin syringe. The pretreatment solution was allowed to reside on the tympanic membrane for 0.5 min prior to dosing.

A small volume (0.3 mL) of 3% moxifloxacin formulation was instilled as a liquid into the region of the tympanic membrane via the external ear with the aid of an otoscope. The liquid formulation, which has a sol-gel transition temperature of approximately 29 to 31°C, gels as its temperature is slowly elevated. A total gelation time of 10 min was allowed before dosing the contralateral ear. The animal's heart rate, respiration rate, body temperature, and depth of anesthesia were continually monitored throughout the procedure.

Following a single dose application of 9 mg (0.3 mL of a formulation containing 30 mg/mL), unbound concentrations of moxifloxacin in the middle ear fluid were monitored using online microdialysis for up to 7200 min post-dose, as described below.

An on-line microdialysis HPLC system was used to quantitate moxifloxacin and ciprofloxacin in the dialysate. Microdialysis perfusion flow rates were controlled with a Harvard microinjection pump (Model H11; Harvard Apparatus Inc.; South Natick, MA) fitted with 5-mL (Hamilton Company, Reno, NV) microsyringes. Microdialysates from both ears were collected alternately into two 25-$\mu$l sample loops of the 10-port valve body controlled by a sequence programmer (Valco Instruments Co. Inc., Houston, TX). The probes were perfused with a retrodialysis calibrator (ciprofloxacin, 5 $\mu$g/mL in PBS) at a flow rate of 0.5 $\mu$L/min.

A Shimadzu 10-A HPLC system (Shimadzu Corporation, Kyoto, Japan) was employed to interface with the Valco on-line sample collection system. It consists of a LC-10AD*vp* pump, a SIL-10A system controller, a CTO-10A column heater, a FCV-10AL*vp* proportioner, and a DGU-14A degasser. A Shimadzu spectrofluorometric detector (RF-10A) with an excitation wavelength of 295 nm and an emission wavelength of 490 nm was also used. A YMC ODS-A 5 $\mu$m, 120 Å (4.6 x 100 mm, Waters Corporation, Milford, MA) column was used for the separation of the compounds and was eluted with a mobile phase composed of 76% ammonium phosphate (20 mM, pH = 2.8) and 24% acetonitrile at a flow rate of 0.5 mL/min. A column temperature of 40°C resulted in retention times of approximately 6 min for moxifloxacin and 3 min for the calibrator, ciprofloxacin.

Example 22-Results of Comparison Between Pretreatment with a 10% Penetration Enhancer and Pretreatment with a 50% Penetration Enhancer

Following external ear dosing of 3% moxigel where the tympanic membranes were pretreated with 10% IPM, unbound moxifloxacin concentrations in the middle ear fluid (Cmef) were typically less that the limit of quantitation. In view of the very few measurable levels observed when pre-treatment utilized 10% IPM, parameters related to delivery of moxifloxacin, could not be calculated.

In contrast, following external ear dosing of 3% moxigel where the tympanic membranes were pretreated with 50% IPM, unbound moxifloxacin concentrations in the middle ear fluid were quite measureable over periods up to 7200 min, or 5 days. In the nine ears studied, Cmax (the maximum unbound moxifloxacin concentrations) in the middle ear fluid (mean $\pm$ SD) was 33.3 $\pm$ 23.3 $\mu$g/mL. Moxifloxacin, which is reported to be 30% to 50% bound to serum proteins in humans, exhibits a total (bound plus free) maximum concentration observed in plasma of 3.1 $\mu$g/mL following an oral dose of 400 mg. The corresponding unbound Cmax in plasma is approximately 1.9 $\mu$g/mL. The mean maximum unbound moxifloxacin concentrations observed in chinchilla middle ear fluid following a single dose of 9 mg into the external ear in the present study is therefore about 15 to 20 times the mean unbound Cmax observed in plasma in humans receiving a 400 mg dose.

Tmax, the time at which maximum concentrations were observed, was 1410 ± 486 min. The AUC from 0 to tlast was about 74,400 ± 52,100 µg-min/mL, and the AUC from 0 to infinity (AUC inf) was approximately 93,400 ± 79,200 µg-min/mL. The mean AUC in healthy humans receiving a single oral dose of 400 mg has been reported to be 2170 µg-min/mL. The corresponding unbound AUC is approximately 60% of this, or about 1300 µg-min/mL. Therefore the mean exposure of the middle ear fluid to unbound levels of moxifloxacin following a single 9-mg dose into the chinchilla external ear is approximately 75 times that seen in the plasma of a healthy human receiving a single oral dose of 400 mg of moxifloxacin.

The extent of trans-tympanic membrane delivery of moxifloxacin into the middle ear fluid was calculated from the mono-exponential concentration-time profiles measured using microdialysis, after dosing moxifloxacin directly into the middle ear space (intrabulla dosing). The volumes of distribution of moxifloxacin in middle ear fluid were estimated to be 1.8 mL. The elimination rate constant was estimated to be 0.0093 min⁻¹. The mean clearance (CL) of moxifloxacin from the middle ear fluid was thus calculated to be 0.0167 mL/min. The extent of delivery (bioavailability, %F) of moxifloxacin into the middle ear fluid was calculated for each data set from the external ear dose, the area under the curve from time 0 to infinity (AUCinf), and the mean clearance from the middle ear fluid (CL) determined from the intrabulla dosing studies, as:

$$\%F = \frac{CL \cdot AUC\,\mathrm{inf}}{Dose} \qquad \text{eq. 1}$$

The fraction of the external ear dose that was delivered to the middle ear space (mean ± SD) was determined to be 17.4 ± 14.7%.

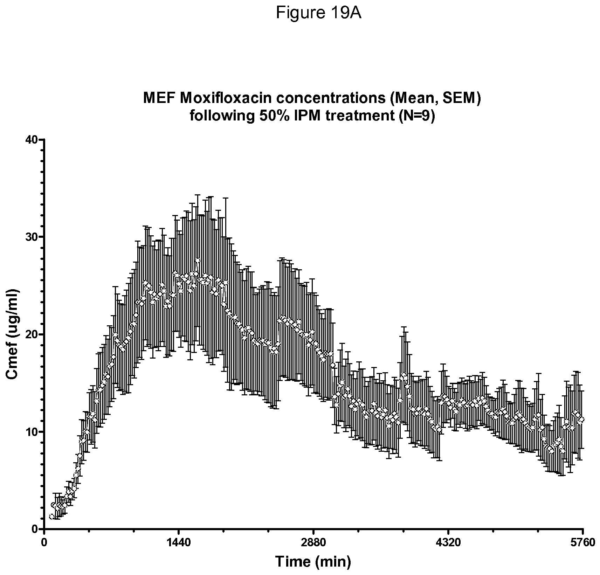

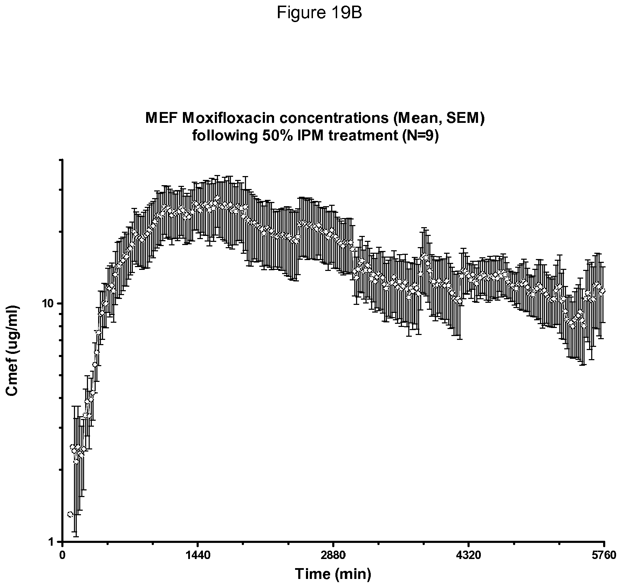

The results of the present studies that examined the transtympanic delivery of moxifloxacin into chinchilla middle ear fluid (MEF) following a dose of 9 mg moxifloxacin, and pretreatment of the tympanic membrane with 50% isopropyl myristate, are shown in Figures 19A and 19B. The corresponding parameters and metrics are shown in Table 10.

Table 10. Delivery of Moxifloxacin Following Pretreatment with a Penetration Enhancer

|  | Mean | SD | %CV |
|---|---|---|---|
| Cmax (µg/mL) | 33.3 | 23.3 | 69.9 |
| Tmax (min) | 1409 | 486 | 34.5 |
| AUClast (min*µg/mL) | 74374 | 52067 | 70.0 |
| AUCinf (min*µg/mL) | 93381 | 79183 | 84.8 |
| Time to reach 10 µg/mL (min) | 618 | 296 | 47.9 |
| Time over 10 µg/mL (min) | 2847 | 1750 | 61.5 |
| %F | 17.4 | 14.7 | 84.8 |

**OTHER EMBODIMENTS**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Claims**

1. A formulation for use by administration to the tympanic membrane,
   wherein said formulation is aqueous and comprises a viscogenic agent and moxifloxacin or a salt thereof;
   wherein said formulation is flowable and has a viscosity less than 100 Pa*s at 25°C;
   wherein said formulation, after application to said tympanic membrane, forms a gel that has a yield stress sufficient to maintain said formulation against said tympanic membrane; and
   wherein said moxifloxacin transfers across the tympanic membrane and into the middle ear space when the formulation is applied to the tympanic membrane.

2. The formulation for use of claim 1, wherein said viscogenic agent is:

   (a) gellan;
   (b) N-isopropyl acrylamide with sodium acrylate and n-N-alkylacrylamide;
   (c) polyacrylic acid with polyethylene glycol;
   (d) polymethacrylic acid with polyethylene glycol;
   (e) CARBOPOL® (polyacrylic acid) with hydroxypropylmethylcellulose;
   (f) cellulose acetate hydrogen phthalate latex; or
   (g) sodium alginate.

3. The formulation for use of claim 1, wherein said viscogenic agent is a reverse thermosetting gel.

4. The formulation for use of claim 3, wherein said viscogenic agent is a poloxamer or a poloxamine.

5. The formulation for use of claim 1, further comprising an anti-inflammatory agent, an anesthetic, an adhesion facilitator, a permeability or penetration enhancer, a bioadhesive, a hygroscopic agent, an ear wax softener, or a preservative.

6. The formulation for use of claim 1, further comprising a penetration enhancer.

7. The formulation for us of claim 6, wherein the penetration enhancer is isopropyl myristate (IPM).

8. The formulation for use of claim 1, wherein the formulation is for use in combination with a penetration enhancer as a pre-treatment, and wherein the penetration enhancer is isopropyl myristate (IPM).

9. An aqueous formulation comprising a viscogenic agent, moxifloxacin or a salt thereof, and isopropyl myristate (IPM), wherein said formulation is flowable and has a viscosity less than 100 Pa*s at 25°C;
   wherein said formulation, after application to the tympanic membrane of the ear of a subject, forms a gel that has a yield stress sufficient to maintain said formulation against the tympanic membrane; and
   wherein said moxifloxacin transfers across the tympanic membrane and into the middle ear space when the formulation is applied to the tympanic membrane.

10. The formulation of claim 9, wherein said viscogenic agent is:

    (a) gellan;
    (b) N-isopropyl acrylamide with sodium acrylate and n-N-alkylacrylamide;
    (c) polyacrylic acid with polyethylene glycol;
    (d) polymethacrylic acid with polyethylene glycol;
    (e) CARBOPOL® (polyacrylic acid) with hydroxypropylmethylcellulose;
    (f) cellulose acetate hydrogen phthalate latex; or
    (g) sodium alginate.

11. The formulation of claim 9, wherein said viscogenic agent is a reverse thermosetting gel.

12. The formulation of claim 11, wherein said viscogenic agent is a poloxamer or a poloxamine.

13. The formulation of claim 9, or the formulation for use according to claim 1, wherein the formulation comprises 10% to 40% viscogenic agent.

14. The formulation of claim 9, or the formulation for use according to claim 1, wherein the formulation comprises 20% to 30% viscogenic agent.

15. The formulation of claim 9, or the formulation for use according to claim 1, wherein the formulation comprises 1% to 3% moxifloxacin or a salt thereof.

**Patentansprüche**

1. Formulierung zur Verwendung durch Verabreichung an das Trommelfell,
   wobei die Formulierung wässrig ist und ein viskogenes Mittel sowie Moxifloxacin oder ein Salz von diesem umfasst;
   wobei die Formulierung fließfähig ist und bei 25°C eine Viskosität von weniger als 100 Pa*s aufweist;
   wobei die Formulierung nach der Applikation auf das Trommelfell ein Gel bildet, das eine ausreichend hohe Formänderungsfestigkeit aufweist, um die Formulierung an dem Trommelfell zu halten; und
   wobei das Moxifloxacin durch das Trommelfell in den Mittelohrraum dringt, wenn die Formulierung auf dem Trommelfell appliziert ist.

2. Formulierung zur Verwendung nach Anspruch 1, wobei es sich bei dem viskogenen Mittel um:

   (a) Gellan;
   (b) N-Isopropylacrylamid mit Natriumacrylat und n-N-Alkylacrylamid;
   (c) Polyacrylsäure mit Polyethylenglykol;
   (d) Polymethacrylsäure mit Polyethylenglykol;
   (e) CARBOPOL ® (Polyacrylsäure) mit Hydroxypropylmethylcellulose;
   (f) Celluloseacetathydrogenphthalat-Latex; oder
   (g) Natriumalginat

   handelt.

3. Formulierung zur Verwendung nach Anspruch 1, wobei es sich bei dem viskogenen Mittel um ein revers thermohärtendes Gel handelt.

4. Formulierung zur Verwendung nach Anspruch 3, wobei es sich bei dem viskogenen Mittel um ein Poloxamer oder ein Poloxamin handelt.

5. Formulierung zur Verwendung nach Anspruch 1, des Weiteren umfassend ein antiinflammatorisches Mittel, ein Anästhetikum, einen Adhäsionsvermittler, einen Permeabilitäts- oder Penetrationsverstärker, eine bioadhäsive Substanz, ein hygroskopisches Mittel, ein Ohrenschmalzenthärtungsmittel oder ein Konservierungsmittel.

6. Formulierung zur Verwendung nach Anspruch 1, des Weiteren umfassend einen Penetrationsverstärker.

7. Formulierung zur Verwendung nach Anspruch 6, wobei es sich bei dem Penetrationsverstärker um Isopropylmyristat (IPM) handelt.

8. Formulierung zur Verwendung nach Anspruch 1, wobei die Formulierung zur Verwendung in Kombination mit einem Penetrationsverstärker als Vorbehandlung vorgesehen ist und wobei es sich bei dem Penetrationsverstärker um Isopropylmyristat (IPM) handelt.

9. Wässrige Formulierung, umfassend ein viskogenes Mittel, Moxifloxacin oder ein Salz von diesem sowie Isopropylmyristat (IPM),
   wobei die Formulierung fließfähig ist und bei 25°C eine Viskosität von weniger als 100 Pa*s aufweist;
   wobei die Formulierung nach der Applikation auf das Trommelfell eines Subjekts ein Gel bildet, das eine ausreichend hohe Formänderungsfestigkeit aufweist, um die Formulierung an dem Trommelfell zu halten; und
   wobei das Moxifloxacin durch das Trommelfell in den Mittelohrraum dringt, wenn die Formulierung auf dem Trommelfell appliziert ist.

10. Formulierung nach Anspruch 9, wobei es sich bei dem viskogenen Mittel um:

    (a) Gellan;
    (b) N-Isopropylacrylamid mit Natriumacrylat und n-N-Alkylacrylamid;
    (c) Polyacrylsäure mit Polyethylenglykol;
    (d) Polymethacrylsäure mit Polyethylenglykol;
    (e) CARBOPOL ® (Polyacrylsäure) mit Hydroxypropylmethylcellulose;
    (f) Celluloseacetathydrogenphthalat-Latex; oder
    (g) Natriumalginat

handelt.

**11.** Formulierung nach Anspruch 9, wobei es sich bei dem viskogenen Mittel um ein revers thermohärtendes Gel handelt.

**12.** Formulierung nach Anspruch 11, wobei es sich bei dem viskogenen Mittel um ein Poloxamer oder ein Poloxamin handelt.

**13.** Formulierung nach Anspruch 9 oder Formulierung zur Verwendung nach Anspruch 1, wobei die Formulierung 10 % bis 40 % des viskogenen Mittels umfasst.

**14.** Formulierung nach Anspruch 9 oder Formulierung zur Verwendung nach Anspruch 1, wobei die Formulierung 20 % bis 30 % des viskogenen Mittels umfasst.

**15.** Formulierung nach Anspruch 9 oder Formulierung zur Verwendung nach Anspruch 1, wobei die Formulierung 1% bis 3 % an Moxifloxacin oder einem Salz von diesem umfasst.

**Revendications**

**1.** Formulation destinée à être utilisée par administration à la membrane tympanique,
ladite formulation étant aqueuse et comprenant un agent viscogène et de la moxifloxacine ou un sel de celle-ci ;
ladite formulation étant apte à l'écoulement et ayant une viscosité inférieure à 100 Pa*s à 25°C ;
ladite formulation formant, après application sur ladite membrane tympanique, un gel qui a une contrainte d'élasticité suffisante pour maintenir ladite formulation contre ladite membrane tympanique ; et
ladite moxifloxacine s'étalant sur la membrane tympanique et pénétrant dans l'espace de l'oreille moyenne lorsque la formulation est appliquée sur la membrane tympanique.

**2.** Formulation à être utilisée selon la revendication 1, dans laquelle ledit agent viscogène est constitué :

(a) de gomme gellane ;
(b) de N-isopropyle acrylamide et d'acrylate de sodium et du n-N-alkylacrylamide ;
(c) d'acide polyacrylique et de polyéthylèneglycol ;
(d) d'acide polyméthacrylique et de polyéthylèneglycol ;
(e) de CARBOPOL® (acide polyacrylique) et de l'hydroxypropylméthylcellulose ;
(f) de latex à base d'acétate de cellulose et de phtalate d'hydrogène ; ou
(g) de l'alginate de sodium.

**3.** Formulation destinée à être utilisée selon la revendication 1, dans laquelle ledit agent viscogène est un gel thermodurcissable inverse.

**4.** Formulation destinée à être utilisée selon la revendication 3, dans laquelle ledit agent viscogène est un poloxamère ou un poloxamine.

**5.** Formulation destinée à être utilisée selon la revendication 1, comprenant en outre un agent anti-inflammatoire, un anesthésique, un facilitateur d'adhérence, un activateur de perméabilité ou de pénétration, un bioadhésif, un agent hygroscopique, un émollient de cérumen ou un conservateur.

**6.** Formulation destinée à être utilisée selon la revendication 1, comprenant en outre un activateur de pénétration.

**7.** Formulation destinée à être utilisée selon la revendication 6, dans laquelle l'activateur de pénétration est le myristate d'isopropyle (IPM).

**8.** Formulation destinée à être utilisée selon la revendication 1, dans laquelle la formulation est destinée à être utilisée en combinaison avec un activateur de pénétration en tant que prétraitement, et dans laquelle l'activateur la pénétration est le myristate d'isopropyle (IPM) .

**9.** Formulation aqueuse comprenant un agent viscogène, de la moxifloxacine ou un sel de celle-ci, et du myristate d'isopropyle (IPM),

ladite formulation étant apte à l'écoulement et ayant une viscosité inférieure à 100 Pa*s à 25°C ;
ladite formulation formant, après application sur ladite membrane tympanique, un gel qui a une contrainte d'élasticité suffisante pour maintenir ladite formulation contre ladite membrane tympanique ; et
ladite moxifloxacine s'étalant sur la membrane tympanique et pénétrant dans l'espace de l'oreille moyenne lorsque la formulation est appliquée sur la membrane tympanique.

10. Formulation selon la revendication 9, dans laquelle ledit agent viscogène est constitué :

(a) de gomme gellane ;
(b) de N-isopropyle acrylamide et d'acrylate de sodium et du n-N-alkylacrylamide ;
(c) d'acide polyacrylique et de polyéthylèneglycol ;
(d) d'acide polyméthacrylique et de polyéthylèneglycol ;
(e) de CARBOPOL® (acide polyacrylique) et de l'hydroxypropylméthylcellulose ;
(f) de latex à base d'acétate de cellulose et de phtalate d'hydrogène ; ou
(g) de l'alginate de sodium.

11. Formulation selon la revendication 9, dans laquelle ledit agent viscogène est un gel thermodurcissable inverse.

12. Formulation selon la revendication 11, dans laquelle ledit agent viscogène est un poloxamère ou une poloxamine.

13. Formulation selon la revendication 9, ou la formulation destinée à être utilisée selon la revendication 1, dans laquelle la formulation comprend 10 à 40% d'agent viscogène.

14. Formulation selon la revendication 9, ou la formulation destinée à être utilisée selon la revendication 1, dans laquelle la formulation comprend 20 à 30% d'agent viscogène.

15. Formulation selon la revendication 9, ou la formulation destinée à être utilisée selon la revendication 1, dans laquelle la formulation comprend 1% à 3% de moxifloxacine ou d'un de ses sels.

Figure 1.  Cumulative percent of moxifloxacin content released *in vitro*

Figure 2:  Response function for deconvolution,
from two intrabulla dose levels (N=9)

Figure 3:  Response function for deconvolution, from high intrabulla dose level (N=3)

## Figure 4A.   Moxifloxacin concentrations in MEF following external ear dosing of 1% moxigel

Cohort 1
- 629R
- 635L
- 635R
- 638R
- 639L
- 640L

Cohort 2
- 640R
- 641L
- 642L
- 643L
- 649L
- 649R

## Figure 4B. Moxifloxacin concentrations in MEF following external ear dosing of 1%moxigel

## Figure 5. Moxifloxacin concentrations (Mean, SD) in MEF following external ear dosing of 1% moxigel (N=23)

## Figure 6.  Moxifloxacin concentrations in MEF following external ear dosing of moxigel 3%

## Figure 7. Moxifloxacin concentrations (Mean, SD) in MEF following external ear dosing of moxigel 3% (N=13)

Figure 8.  Moxifloxacin concentrations (Mean, SD) in MEF following external ear dosing of 1% and 3% moxigel

Figure 9. Comparison of Cmax and Tmax for 1% and 3% moxigel

Figure 10. Cumulative amount delivered into MEF following external ear dosing of 1% moxigel (N=23)

# Figure 11A. Input rate into MEF following external ear dosing of 1% moxigel

Input rate, 1st cohort, N=6

Input rate, 2nd cohort, N=6

## Figure 11B. Input rate into MEF following external ear dosing of 1% moxigel

Input rate, 3rd cohort, N=6

Input rate, 4th cohort, N=5

Figure 12. Cumulative amount delivered into MEF following external ear dosing of 3% moxigel (N=13)

# Figure 13.  Input rate into MEF following external ear dosing of 3% moxigel

Input rate, 1st cohort, N=6

Input rate, 2nd cohort, N=7

## Figure 14. Input rate into MEF following external ear dosing of 1% and 3% moxigel

**Figure 15. Time to reach and duration above target concentrations in the middle ear fluid following external ear dosing with 1% moxigel**

**Figure 16. Time to reach and duration above target concentrations in the middle ear fluid following external ear dosing with 3% moxigel**

Time to reach target Cmef

time to reach 10 ug/ml
time to reach 20 ug/ml

median = 180 min

median = 240 min

Time (min)

Time above target Cmef

median = 2740 min

median = 2420 min

time above 10 ug/ml
time above 20 ug/ml

Time (min)

Figure 17. AUIC in MEF over 3 days following external ear dosing

## Figure 18.  Cmax/MIC in MEF following external ear dosing

Figure 19A

**MEF Moxifloxacin concentrations (Mean, SEM)
following 50% IPM treatment (N=9)**

Figure 19B

**MEF Moxifloxacin concentrations (Mean, SEM)
following 50% IPM treatment (N=9)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009142719 A
- US 6201072 B

**Non-patent literature cited in the description**

- Poloxamers in the Pharmaceutical Industry. **IRVING R. SCHMOLKA.** Polymers for Controlled Drug Delivery. 1990
- **JOSSART et al.** *Pharm. Res.,* 1990, vol. 7, 1242-7
- **HUANG et al.** *J. Pharm. Sci.,* 2001, vol. 90, 2088-98
- Pharmacodynamics of Quinolones. **OWENS ; AMBROSE.** Antimicrobial Pharmacodynamics in Theory and Clinical Practice. Marcel Dekker, 2002, 162
- **SCHENTAG et al.** *Ann. Pharmacother.,* 2003, vol. 37, 1287-98
- **SCHENTAG et al.** *Ann. Pharmacotherap.,* 2003, vol. 37, 1478-88